(19)

Europäisches
Patentamt
European
Patent Office
Office européen
des brevets

(11)    **EP 2 542 148 B1**

(12)    **EUROPEAN PATENT SPECIFICATION**

(45) Date of publication and mention
of the grant of the patent:
**30.05.2018  Bulletin 2018/22**

(51) Int Cl.:
***A61B 5/02*** *(2006.01)*

(21) Application number: **11751440.6**

(86) International application number:
**PCT/US2011/027237**

(22) Date of filing: **04.03.2011**

(87) International publication number:
**WO 2011/109734 (09.09.2011 Gazette 2011/36)**

(54) **ACTIVE PHYSICAL PERTURBATIONS TO ENHANCE INTELLIGENT MEDICAL MONITORING**

AKTIVE PHYSIKALISCHE STÖRUNGEN ZUR VERSTÄRKUNG EINER INTELLIGENTEN MEDIZINISCHEN ÜBERWACHUNG

PERTURBATIONS PHYSIQUES ACTIVES POUR AMÉLIORER LA SURVEILLANCE MÉDICALE INTELLIGENTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2010  US 310583 P**

(43) Date of publication of application:
**09.01.2013  Bulletin 2013/02**

(73) Proprietors:
• **The Regents of the University of Colorado, a body corporate**
**Denver, CO 80203 (US)**
• **Flashback Technologies, Inc.**
**Boulder, CO 80301 (US)**

(72) Inventors:
• **GRUDIC, Gregory Zlatko**
**Longmont, Colorado 80503 (US)**
• **MOULTON, Steven Lee**
**Littleton, Colorado 80121 (US)**
• **MULLIGAN,, Isobel Jane**
**Niwot, CO 80503 (US)**

(74) Representative: **V.O.**
**P.O. Box 87930**
**2508 DH Den Haag (NL)**

(56) References cited:
WO-A2-03/077854          WO-A2-2007/149533
US-A1- 2006 178 585      US-A1- 2007 015 972
US-A1- 2007 213 619      US-B1- 7 654 964

• W. H. COOKE: "Lower body negative pressure as a model to study progression to acute hemorrhagic shock in humans", JOURNAL OF APPLIED PHYSIOLOGY, vol. 96, no. 4, 1 April 2004 (2004-04-01), pages 1249-1261, XP055083116, ISSN: 8750-7587, DOI: 10.1152/japplphysiol.01155.2003
• P. LAMBERT ET AL: "Does a positive end-expiratory pressure-induced reduction in stroke volume indicate preload responsiveness? An experimental study", ACTA ANAESTHESIOLOGICA SCANDINAVICA, vol. 51, no. 4, 1 April 2007 (2007-04-01), pages 415-425, XP55083136, ISSN: 0001-5172, DOI: 10.1111/j.1399-6576.2007.01248.x
• K. L. RYAN ET AL: "Breathing through an inspiratory threshold device improves stroke volume during central hypovolemia in humans", JOURNAL OF APPLIED PHYSIOLOGY, vol. 104, no. 5, 21 February 2008 (2008-02-21), pages 1402-1409, XP55083125, ISSN: 8750-7587, DOI: 10.1152/japplphysiol.00439.2007

**Description**

**STATEMENT AS TO RIGHTS TO INVENTIONS MADE UNDER FEDERALLY SPONSORED RESEARCH OR DEVELOPMENT**

The United States Federal Government may have rights to this invention pursuant to DOD Grant No. W81XWH-09-C-1060.

**COPYRIGHT STATEMENT**

A portion of the disclosure of this patent document contains material that is subject to copyright protection. The copyright owner has no objection to the facsimile reproduction by anyone of the patent document or the patent disclosure as it appears in the Patent and Trademark Office patent file or records, but otherwise reserves all copyright rights whatsoever.

**FIELD**

The present disclosure relates, in general, tools and techniques for medical monitoring, and more particularly, to solutions that employ, take advantage of, and/or measure physical perturbation of the body to enhance such monitoring.

**BACKGROUND**

Medical monitoring is an important diagnostic tool in a variety of fields of medicine. For example, in the areas of traumatic brain injury ("TBI"), hemorrhage, and the like, the ability of a medical practitioner to assess the patient's current condition, and predict the patient's future condition, can mean the difference between life and death. Unfortunately, conditions such as these can be difficult to assess in real time, without continuous, invasive and/or non-invasive monitoring.

US2006/0178585 discloses an apparatus for assessing a cardiovascular system of a mammal comprising: a cuff for occluding systolic blood flow through a brachial artery of the mammal; a transducer having an electronic output representing a mechanical waveform in tissue subject to a blood flow occlusion by said cuff; and a computing device receiving said output and analyzing a temporal and amplitude variation thereof, generating a value representing a compliance of an aorta of the mammal. The computing device analyzes a change in compliance with respect to differing conditions of the mammal.

US2007/0213619 provides a method of detecting the presence of a baroreflex response. According to the document, in one embodiment, a method of quantifying a baroreflex response includes: obtaining photoplethysmographic data; determining an instantaneous value and a sustained value for one or more of the following parameters: (a) a normalized peak width of each cardiac cycle; (b) an amplitude of the pulsatile component of each cardiac cycle in the PPG data derived from an ear sensor; and (c) an instantaneous pulse rate; determining a ratio of the instantaneous value to the sustained value for one or more of (a), (b) and (c); and comparing the ratio to a library of known ratios.

Techniques have been developed to allow for enhanced monitoring, including the use of continuous non-invasive monitoring of certain physiological parameters to estimate and/or predict physiological symptoms. For example, International Publication No. WO/2010/053743 A1, filed by Grudic et al. on October 26, 2009 (the "'743 Publication"), describes several techniques for employing a model to analyze, and learn from, continually changing data sets, such as blood pressure waveform data. Through the use of such models, practitioners can estimate physiological conditions, such as hemmorrhage, cardiovascular collapse, and/ the like, from easily measurable data, such as blood pressure, pulse oximetry, and the like.

Often, however, such models may require refinement and/or adjustment in order to provide optimal predictive characteristics for a particular patient. Unfortunately, such refinement often requires the patient (or test subjects) to undergo an actual (or experimental) significant physiological event in order to obtain the data with which to construct and/or refine the model. Thus, there is a need for tools and techniques that can enable the construction and/or modification of such models without the need to expose test subjects to significant physiological events.

**BRIEF SUMMARY**

The invention is defined by the features of independent claims 1 and 24. A set of embodiments provides tools and techniques for enhancing intelligent medical monitoring, and in particular monitoring that employs models for estimating and/or predicting physiological conditions. In an aspect, some of these tools and techniques employ active physical perturbation of a test subject (or patient), to induce physiological changes in the subject. By monitoring one or more of the patient's physiological parameters (e.g., non-invasively monitoring the subject's blood pressure) shortly before, during and/or after the physical perturbation, the subject's reaction to the perturbation can be determined, and this reaction can be used to better estimate and/or predict the subject's physiological state and/or clinical condition. In a particular case, the subject's response to the physical perturbation can be used to construct and/or refine a model that can be applied to analyze the subject's physiological parameters to produce such predications and/or estimations.

The tools provided by various embodiments include, without limitation, methods, systems, and/or software products. Merely by way of example, a method might comprise one or more procedures, any or all of which are executed by a computer system. Correspondingly, an embodiment might provide a computer system configured with instructions to perform one or more procedures in accordance with methods provided by various other embodiments. Similarly, a computer program might comprise a set of instructions that are executable by a computer system (and/or a processor therein) to perform such operations. In many cases, such software programs are encoded on physical, tangible and/or non-transitory computer readable media (such as, to name but a few examples, optical media, magnetic media, and/or the like).

For example, one set of embodiments provides methods. An exemplary method of predicting a subject's future physiological state, or estimating the current physiological state, might comprise applying lower body negative pressure ("LBNP") to the subject for an amount of time sufficient to cause variation in a blood pressure of the subject but insufficient to provoke cardiovascular collapse in the subject. In an aspect of some embodiments, the method further comprises monitoring continuous blood pressure waveform data of the subject using a non-invasive monitor. This monitoring can include recording a plurality of blood pressure readings of the subject, which can comprise a first set of one or more blood pressure readings prior to application of the lower body negative pressure and a second set of one or more blood pressure readings during and/or after application of the lower body negative pressure.

In some cases, the method additionally comprises analyzing, with a computer system, the blood pressure waveform data against a pre-exiting model, and/or generating, e.g., based on analysis of the blood pressure waveform data, an estimated of blood loss volume of the subject, and/or an estimated volume of blood loss at which the subject will suffer cardiovascular collapse. In other cases, the method might comprise displaying information about the predicted volume of blood loss at which the subject will suffer cardiovascular collapse.

An exemplary method in accordance with another set of embodiments comprises physically perturbing a body of a patient with one or more physical perturbations. The method, in some cases, might further comprise receiving, at a computer system, a set of input data from one or more physiological sensors, the set of input data pertaining to one or more physiological parameters of the physically perturbed body of the patient. The computer system might analyze the input data to determine a patient response to the one or more physical perturbations, and the method might further comprise generating (e.g., with the computer system) diagnostic data concerning one or more physiological states of the patient. The method can additionally comprise displaying, e.g., with a display device, at least a portion of the diagnostic data.

An apparatus, in accordance with yet another set of embodiments, might comprise a computer readable medium having encoded thereon a set of instructions executable by one or more computers to perform one or more operations. In some embodiments, the set of instructions might comprise instructions for performing some or all of the operations of methods provided by certain embodiments.

Merely by way of example, in one embodiment, the set of instructions comprises instructions for receiving, at a computer system, a set of input data from one or more physiological sensors, the set of input data pertaining to one or more physiological parameters of a physically perturbed body of a patient; instructions for analyzing, with the computer system, the input data to determine a patient response to the one or more physical perturbations; and/or instructions for generating diagnostic data concerning one or more physiological states of the patient. The set of instructions might further comprise instructions for displaying, with a display device, at least a portion of the diagnostic data.

A system, in accordance with yet another set of embodiments, might comprise one or more processors and a computer readable medium in communication with the one or more processors. The computer readable medium might have encoded thereon a set of instructions executable by the computer system to perform one or more operations, such as the set of instructions described above, to name one example. In some embodiments, the system might further comprise a perturbation device and/or a therapeutic device, either or both of which might be in communication with the processor and/or might be controlled by the processor. Merely by way of example, the perturbation device might be configured to physically perturb the body of the patient with one or more physical perturbations.

## BRIEF DESCRIPTION OF THE DRAWINGS

A further understanding of the nature and advantages of particular embodiments may be realized by reference to the remaining portions of the specification and the drawings, in which like reference numerals are used to refer to similar components. In some instances, a sub-label is associated with a reference numeral to denote one of multiple similar components. When reference is made to a reference numeral without specification to an existing sub-label, it is intended to refer to all such multiple similar components.

Fig. 1 is a schematic diagram illustrating a basic structure for various embodiments.

Fig. 2 is a process flow diagram illustrating a method of enhancing medical monitoring using physical perturbations.

Fig. 3 is a process flow diagram illustrating a method of generating a model of a physiological state.

Fig. 4 is a process flow diagram illustrating a method of correlating physiological data with a measured physiological state of a subject.

Fig. 5 is a generalized schematic diagram illustrating a computer system, in accordance with various embodiments.

## DETAILED DESCRIPTION OF CERTAIN EMBODIMENTS

While various aspects and features of certain embodiments have been summarized above, the following detailed description illustrates a few exemplary embodiments in further detail to enable one of skill in the art to practice such embodiments. The described examples are provided for illustrative purposes and are not intended to limit the scope of the invention.

In the following description, for the purposes of explanation, numerous specific details are set forth in order to provide a thorough understanding of the described embodiments. It will be apparent to one skilled in the art, however, that other embodiments of the present may be practiced without some of these specific details. In other instances, certain structures and devices are shown in block diagram form. Several embodiments are described herein, and while various features are ascribed to different embodiments, it should be appreciated that the features described with respect to one embodiment may be incorporated with other embodiments as well. By the same token, however, no single feature or features of any described embodiment should be considered essential to every embodiment of the invention, as other embodiments of the invention may omit such features.

Unless otherwise indicated, all numbers used herein to express quantities, dimensions, and so forth should be understood as being modified in all instances by the term "about." In this application, the use of the singular includes the plural unless specifically stated otherwise, and use of the terms "and" and "or" means "and/or" unless otherwise indicated. Moreover, the use of the term "including," as well as other forms, such as "includes" and "included," should be considered non-exclusive. Also, terms such as "element" or "component" encompass both elements and components comprising one unit and elements and components that comprise more than one unit, unless specifically stated otherwise.

One set of embodiments provides tools and techniques for enhancing intelligent medical monitoring, and in particular monitoring that employs models for estimating and/or predicting physiological state or clinical condition. In an aspect, some of these tools and techniques employ active physical perturbation of a test subject (or patient), to induce physiological changes in the subject. By monitoring one or more of the patient's physiological parameters (e.g., non-invasively monitoring the subject's blood pressure) shortly before, during, and/or after the physical perturbation, the subject's reaction to the perturbation can be determined, and this reaction can be used to estimate and/or predict the subject's physiological state and/or clinical condition. In a particular case, the subject's response to the physical perturbation can be used to construct and/or refine a model that can be applied to analyze the subject's physiological parameters to produce such predications and/or estimations.

Merely by way of example, in some cases, the body of a subject (which might be a patient, a test subject, etc.) can be physically perturbed (e.g., a positive or negative pressure breath delivered by a ventilator, using an impedance threshold device to affect airway pressure, a lower body negative pressure chamber to divert central blood volume to the pelvis and lower extremities, etc.). That perturbation often will affect one or more of the subject's physiological parameters (e.g., blood pressure, pulse oximetry, etc.). Some embodiments can determine the effect of the perturbation on the measured physiological parameters (e.g., by collecting waveform data and comparing samples of the data taken before the perturbation with samples taken during or after the perturbation). The effect of the perturbation can be analyzed against an analytical model to allow a correlation between the effect of the perturbation and a current or future physiological state or clinical condition of the subject. The effect of the perturbation can also be measured to estimate and/or predict clinical conditions such as hemorrhage, cardiovascular collapse, and/or the like. Alternatively and/or additionally, if the subject actually experiences the physiological state, the physiological parameters of the subject can be monitored at that time and/or compared with the predictions about the conditions under which the subject would experience that state; the model, then can be updated to comport with this additional data.

A number of analytical models can be employed in accordance with various embodiments. Merely by way of example, the '743 Publication, describes techniques for generating and employing an analytical model that can be used to enhance medical monitoring in accordance with various embodiments. Other techniques that can be used alternatively and/or additionally include, without limitation, support vector machines, as described in Scholkopf, B., & Smola, A. (2002), Learning with Kernels, Cambridge, MA: MIT Press; the Bayesian methods described in Wainwright, M. J., & Jordan, M. I. (2008), "Graphical Models, Exponential Families, and Variational Inference," Foundations and Trends® in Machine Learning, 1 (1-2), 1-305; minimax probability machines, as described in Strohmann, T., & Grudic, G. (2003), "A formulation for minimax probability machine regression," Advances in Neural Information Processing Systems 15 (pp. 769-776), MIT Press, Cambridge, MA, Strohmann, T., Belitski, A., Grudic, G., & DeCoste, D. (2004), "Sparse greedy minimax probability machine classification," Advances in Neural Information Processing Systems 16, MIT Press, Cambridge, MA, and Bohte, S., Breitenbach, M., & Grudic, G. (2004), "Nonparametric classifcation with polynomial mpmc cascades," International Conference on Machine Learning (ICML); the clustering algorithms described in Breitenbach, M., & Grudic, G. (2005), "Clustering through ranking on manifolds," International Conference on Machine Learning (ICML); and other machine learning algorithms, such as those described in Hastie, T., Tibshirani, R., & Friedman, J. (2009), The Elements of Statistical Learning: Data Mining. Inference, and Prediction (Vol. 2), Springer-Verlag, Ripley, B. D. (1996), Pattern Recognition and Neural Networks, Cambridge University Press, and Schapire, R. E., Freund, Y., Bartlett, P., & Lee, W. S. (1998), "Boosting the margin: A new explanation for the effectiveness of voting methods," Annals of Statistics, 26 (6), 1651-1686.

Using such techniques, a model can be constructed, in a general sense, by physically perturbing the body of a subject until a specified physiological state has been attained, while measuring one or more of the subject's physiological parameters throughout the course of the perturbation. The conditions of the perturbation, and/or the subject's physiological parameters and/or clinical condition(s), at (or immediately before) the occurrence of the specified physiological state can be correlated, e.g., using the machine learning techniques in the references described above, with the subject's physiological parameters at an earlier stage of the perturbation. This process can be repeated with a sufficient number of trials (with the same or different subjects) to identify, with statistical significance, which one or more of several physiological parameters have a predictive effect on the physiological state and/or clinical condition(s). In some cases, the physiological state may be irreversible and/or terminal, in which case the subjects (i.e., animal subjects) may have to be sacrificed after the experiment. In other cases, the physiological state may be recoverable, in which case human or animal subjects may be used.

From this model, the susceptibility of a different subject to the physiological state (and/or under what conditions that subject will experience that physiological state) can be predicted, as a general matter, from measurements of the change in the subject's physiological parameters to a milder form of the perturbation than that which caused the physiological state to occur in the test subjects.

This modeling technique has application in a wide variety of fields, and a number of different physical perturbations may be used to build the model and/or analyze a patient's physiological parameters against such a model. (It should be noted that the same mode of physical perturbation need not necessarily be used to induce the physiological state in the test subjects, on the one hand, and enhance the medical monitoring of a patient, on the other hand, so long as a correlation can be drawn between changes induced to the subject's physiological parameters in response to each of the different modes of perturbation.)

Merely by way of example, one mode of physically perturbing a subject's body is to apply lower body negative pressure ("LBNP") to the body, using a LBNP chamber. Another mode of perturbation is to induce pressure changes (i.e., either positive pressure or negative pressure, or both) to the subject's airway or other body parts. One way to induce such pressure changes to the airway is through an impedance threshold device. An impedance threshold device ("ITD"), in general, can be any device that restricts inspiratory airflow, which can result in negative intrathoracic pressure, an increase in venous return to the heart, and/or an increase in stroke volume/blood flow/circulation. An intrathroacic pressure regulating device ("ITPR") can be considered a type of ITD, in that it functions in a similar way, on ventilated patients, by introducing an active vacuum source to the thorax through the respiratory circuit. The RESQGARD™ and RESQPOD™ devices, both available from Advanced Circulatory Systems, Inc., are examples of ITD devices that can be used to physically perturb a body, in accordance with certain embodiments.

Other devices, such as blood pressure cuffs and the like, can be used to induce positive pressure to desired areas of the body. Likewise, various techniques that might not require any additional equipment, can be used to perturb the body. For example, if the desired perturbation is to induce positive pressure on the subject's airway, a valsalva maneuver might be performed. Alternatively and/or additionally, positive pressure can be induced on an airway during spontaneous, unassisted breathing, or positive pressure can be induced on an airway during mechanical ventilation, e.g., through use of a ventilator.

Other types of physical perturbations are possible as well. Merely by way of example, in some cases, the delivery of medication and/or fluids (e.g., intravenously or otherwise) can serve as a physical perturbation. In other cases, the body may be perturbed through delivery of electrical current to the body of the subject (or a desired portion thereof, e.g., using an automatic implantable cardioverter defibrillator ("AICD"). More generally, a physical perturbation of the subject's body can be an action that imposes a physical change or stress on a subject's body (or a portion thereof) that results in a measurable change in a physiological parameter, such that the change can be used to enhance the medical monitoring process, or more particularly, facilitate the estimation and/or prediction of a physiological state of the subject.

Sometimes, the body will perturb itself such as a change in position (e.g., sitting or standing up), taking a spontaneous breath, or when there is a premature ventricular contraction (PVC) of the heart. Position changes can impact the cardiovascular system, leading to increasing or decreasing venous return and/or a rise or fall in blood pressure. A spontaneous breath leads to greater negative intrathoracic pressure, an increase in venous return, greater filling of the heart, augmentation of cardiac stroke volume and/ or a change in blood pressure. PVC's cause compensatory pauses in the cardiac cycle, which leads to greater filling of the heart, augmentation of the stroke volume and/or a change in blood pressure. These various perturbations of the cardiopulmonary system can be monitored and the body's response measured to continuously estimate the physiological state and/or clinical condition of the patient.

As used herein, the term, "physiological state" means any state or condition that a subject (e.g., a patient) may experience. In a variety of embodiments, a particular physiological state may be of interest, and/or might have adverse long-term health consequences for the subject, such that an estimation and/or prediction of that physiological state may be helpful in diagnosing and/or treating the subject. A given physiological state might be defined and/or diagnosed qualitatively and/or quantitatively, and can include any of a variety of adverse conditions. Examples of physiological states can include, without limitation, clinical conditions such as blood loss volume, a blood loss volume that results in hemodynamic decompensation, congestive heart failure, cardiogenic shock, cardiac tamponade, valvular heart disease, intracranial hypertension, neurogenic shock, intracranial pressure, seizure, sepsis, tension pneumothorax, and a variety of other conditions.

As used herein, the term "physiological parameter" means any measurable aspect of a subject's (e.g., a patient's) physiology, and can include, a variety of physiological data. Merely by way of example, physiological data can be received (e.g., input) from a physiological sensor that is measuring one or more physiological parameters of a patient. Physiological feature data can then be derived from the physiological data. For example, a Finometer continuous non-invasive blood pressure monitor (physiological sensor) can be used to measure the blood pressure of a patient and provide blood pressure waveform data (physiological data). From the blood pressure data, blood volume data (physiological feature data) can be derived. Various other physiological feature data can be derived from the physiological data. From the physiological feature data, other physiological parameters (e.g., parameters not amenable to rapid, noninvasive, direct measurement) can be estimated/predicted, and/or a prediction can be made about a physiological threshold where a given physiological state is reached (e.g., trauma, sepsis, or cardiogenic shock). The prediction can be based on a large data set of physiological feature data. Moreover, the prediction can use any type of predictive algorithm and/or can be self-learning. In some embodiments, a user interface can provide the physiological feature data along with the predicted threshold. Such a user interface can allow a user to determine whether the physiological feature data is converging and/or diverging with the threshold data.

Moreover, a variety of other physiological parameters and/or sensors can be used to develop models and/or to estimate/predict various physiological states based on those models. Merely by way of example, a blood pressure sensor, an intracranial pressure monitor, a central venous pressure monitoring catheter, an electroencephalograph, a cardiac monitor, a transcranial Doppler sensor, a transthoracic impedance plethysmograph, a pulse oximeter, a near infrared spectrometer, a ventilator, an electronic stethoscope, an accelerometer, an electrooculogram, and a transcutaneous glucometer and/or electrolyte sensor are but a few examples of sensors that can be used, inter alia, to measure physiological parameters before, during, and/or after exposure of a subject to a physical perturbation.

The electrocardiograph ("ECG") measures the heart's electrical activity using specifically placed electrodes. The output describes cardiac muscle activity through voltages along different directions between electrode pairs. The typical ECG waveform is described as a P wave, a QRS complex, and a T wave. Obviously heart rate ("HR") data can be extracted from the waveform, and considerable attention has been given to heart rate variability ("HRV") for evaluating autonomic dysfunction, and its correlation to events such as increased intracranial pressure and death due to traumatic injury. Some scholars have found that the performance of HRV for predicting traumatic head injury was improved by considering factors such as heart rate, blood pressure, sedation, age, and gender. There are various algorithmic definitions for computing HRV from R-R intervals, which appear to perform equivalently as long as they are calculated over extended (5 min) intervals. ECG poses some challenges for usability in transport as the motion of subjects can alter readings or dislodge sensors.

In their basic form, pulse oximeters use the differing properties of deoxygenated and oxygenated hemoglobin for absorbing red and infrared light. Red ("R") and infrared ("IR") LEDs shine through a relatively translucent site such as the earlobe or finger, and a photodetector on the other side receives the light that passes through. The observed values are used to compute the ratio R/IR, which can be used to look up the patient's saturation of peripheral oxygen ($SpO_2$) level from pre-computed tables. As the heart beats, blood pulses through the arteries in the measurement location causing more light to be absorbed, yielding a waveform of light signals over time. This photoplethysmograph ("PPG") waveform can be used to determine heart rate, but also analyzed in its own right. Subtracting the trough (DC) values, which represent constant light absorbers, we are left with the absorption properties for the varying (AC) component, which is arterial blood. Advances in technology have seen more than 30 light wavelengths used to make systems more

reliable by distinguishing $O_2$ and $CO_2$.

Some research recommends the use of the raw PPG signal, and discusses its relationship to systolic blood pressure, sympathetic tone and respiration. PPG has been shown to be correlated to systolic pressure variation ("SPV"), which, in turn, is correlated with hypovolemia. Other researchers compare correlation of ear and finger pulse oximeter waveforms to SBP. They evaluate pulse amplitude, width and area under the curve as extracted features, and use metrics on the envelope of the PPG waveform to reliably detect blood sequestration of more than 1 liter induced by lower body negative pressure ("LBNP"). Still others have constructed a linear predictor for cardiac output ("CO"), based on heart rate and features extracted from the ear PPG waveform.

The Perfusion Index ("PI") expresses the varying versus stationary components of IR light in the PPG as a percentage:

$$PI = \frac{AC_{IR}}{DC_{IR}} \times 100\% \qquad \text{(Eq. 1)}$$

Research has established the correlation of PI and core-to-toe temperature difference (a measure of peripheral perfusion) for critically ill patients.

The Pleth Variability Index ("PVI") describes changes in PI over at least one respiratory cycle:

$$PVI = \frac{PI_{max} - PI_{min}}{PI_{max}} \times 100\% \qquad \text{(Eq. 2)}$$

It has been shown that PVI can predict fluid responsiveness in anesthetized and ventilated patients. Moreover, PPG variation, pulse pressure variation ("PPV"), and systolic pressure variation ("SPV") are well correlated to gradual autodonation to a reduction of 20% in systolic blood pressure.

The Finopres system, which is included in the U.S. Army Institute of Surgical Research ("USAISR") lower body negative pressure protocol, uses a volume clamp mechanism to measure the finger arterial pressure waveform, as well as estimate parameters such as CO, and stroke volume ("SV"). The mechanism combines an infrared plethysmograph to determine baseline unloaded artery diameter and monitor blood volume, and an inflatable finger cuff which is controlled to maintain baseline diameter. Variation in cuff pressure provides an indirect means of measuring intra-arterial pressure.

Similar parameters can be obtained using ICG, which measures volumetric changes due to the cardiac cycle by observing changes in thoracic impedance. Current is passed through the chest between sensors, traveling through the aorta as the path of least resistance. As blood velocity and volume change in the aorta, corresponding changes in impedance are recorded as a continuous waveform, from which hemodynamic parameters such as CO and SV can be computed.

Many standard hemodynamic parameters intended to capture the behavior of the cardiac cycle are derived from blood pressure and heart rate measurements. For example, arterial blood pressure ("ABP") is the pressure in the arteries, which varies through the systolic and diastolic phases of the cardiac cycle. Systolic blood pressure ("SBP") is the maximum ABP as the left ventricle contracts. It can be extracted as the peak values of the raw Finopres ABP waveform. Diastolic blood pressure (DBP) is the ABP when the heart is at rest. It can be measured from the troughs of the APB waveform. Mean arterial pressure ("MAP") describes the mean arterial blood pressure over a cardiac cycle, and it can be expressed as

$$MAP = (CO \times SVR) + CVP \qquad \text{(Eq. 3)}$$

where CO is cardiac output, SVR is systemic vascular resistance and CVP is central venous pressure. MAP can be approximated using more accessible parameters as:

$$MAP \cong DPB + \frac{1}{2}(SBP - DBP) \qquad \text{(Eq. 4)}$$

Systolic pressure variability ("SPV") attempts to measure the change or variability in SBP over a respiration cycle. In general, it is expressed as the difference (or percentage change) between min and max SBP:

$$SPV = SBP_{\max R} - SBP_{\min R} \qquad \text{(Eq. 5)}$$

Authors will also frequently distinguish delta up ("dUp") and delta down ("dDown") components. Researchers have examined the correlation between SPV and dDown for hemorrhage and volume replacement, finding that they followed intravascular volume for mechanically ventilated patients. Others have concluded that dDown was an effective indicator of CO response to volume replacement for mechanically ventilated septic shock patients. One researcher points out that SPV and dDown should be calculated as percentages of SBP in the case of hypotension.

Pulse pressure (PP) is the beat to beat change in blood pressure:

$$PP = SBP - DBP \qquad \text{(Eq. 6)}$$

Pulse pressure variability (PPV) is also computed using min and max PP over the respiratory cycle:

$$PPV = PP_{\max R} - PP_{\min R} \qquad \text{(Eq. 7)}$$

It has been shown that higher PPV percentages indicated which patients in septic shock responded to fluids and also demonstrated a correlation between PPV and cardiac index. Others have concluded that PPV can be an effective measure for fluid management.

Stroke volume ("SV"), or volume of blood pumped by the left ventricle in a single contraction, is the difference between the volume of blood in the ventricle at the end of the diastolic phase minus the volume of blood remaining after the heart beat:

$$SV = end\_diastolic\_volume - end\_systolic\_volume \qquad \text{(Eq. 8)}$$

Since these constituent parameters are difficult to measure, SV is generally estimated from the ABP waveform, and it has been shown that SV and PP derived from Finometer BP estimates are correlated with blood loss.

Cardiac Output ("CO") is the volume of blood pumped per unit time:

$$CO = SV \times HR \qquad \text{(Eq. 9)}$$

Cardiac index ("CI") relates the performance of the heart to the size of the patient using body surface area ("BSA"):

$$CI = \frac{CO}{BSA} \qquad \text{(Eq. 10)}$$

BSA can be estimated using height and mass of the individual. It has been found that CI and mixed venous oxygen saturation showed a linear relationship to blood loss.

Near infrared spectroscopy ("NIRS") has been used for measuring tissue oxygenation since the 1970's. NIR light is shone on the body and deeply penetrates skin, fat and other layers, where it is either scattered or absorbed. As with pulse oximeters the differing absorption characteristics of oxyhemoglobin ("$O_2Hb$") and deoxyhemoglobin ("HHb") are used to calculate concentrations based on light received by a detector. Other parameters such as pH and hematocrit can also be extracted from the spectra. Research has modified this process to compensate for the interference of skin and fat layers to better measure muscle oxygen saturation ("$SmO_2$"), and NIRS measurements of $SmO_2$ and pH have been tested as indicators of hemodynamic instability with subjects undergoing LBNP and conclude that $SmO_2$ is an early indicator of vasoconstriction and impending hemodynamic decompensation. Others have compared NIRS forearm measurements of $SmO_2$ and muscle oxygen tension ("$PmO_2$") to $StO_2$ measured at the thenar eminence with a commercial device. They concluded that spectroscopic observations of $PmO_2$ and $SmO_2$ were early indicators of hemodynamic decompensation due to LBNP, while thenar $StO_2$ did not change throughout the test.

Other noninvasive sensors offer different system measurements which may contribute a new view to the techniques described herein. Transcranial Doppler uses sound waves (a pulsed Doppler probe) to measure blood flow velocities in cerebral blood vessels (cerebral blood flow ("CBF") velocity). It poses challenges in determining recording locations with a clear path to the vessels of interest, but some have used CBF velocities as an indicator for dynamic cerebral autoregulation under hypervolemia with hemodilution.

The respiration cycle is intimately related to the cardiac cycle and may offer relevant measurements for the problem. A capnogram measures the concentration of $CO_2$ in respiratory gases and is an indirect measure of the $CO_2$ in arterial blood. Infrared light is passed through the gas sample, where $CO_2$ absorbs it and a detector on the other side observes this decrease in light. End tidal $CO_2$ ("$EtCO_2$"), or the $CO_2$ concentration at the end of exhalation, has been determined to have a logarithmic relationship to cardiac output and has been found to track SV in an LBNP model for progressive central hypovolemia, but that the decreases were small relative to baseline measurements for subjects, and concluded that sensors providing continuous trending were required. Other features extracted from sensors include the nonlinear entropy-based features for biosignal analysis.

## Exemplary Use Cases

The techniques provided by various embodiments can be used to enhance monitoring of a variety of medical and surgical conditions. Merely by way of example, in an exemplary embodiment, a model of cardiovascular collapse was generated using data from 150 human test subjects, aged 18-55. Each subject was monitored with a non-invasive blood pressure sensor while placed in a lower body negative pressure ("LBNP") chamber. A vacuum was applied to the lower body (i.e., physically perturbing the subject's body), in a step-wise fashion, until the subject experienced cardiovascular collapse (marked by bradycardia and hypotension). At that point, lower body negative pressure was released, sequestered blood volume was redistributed to the entire body, and the subject recovered without long-term harm to the subject. After repeated trials with different subjects, it was discovered, using the analytical techniques described in the '473 Publication, that the subject's reaction (as indicated by the noninvasive blood pressure sensor) to the first step in the LBNP process was an accurate predictor of the LBNP level at which the subject would collapse, and a model of cardiovascular collapse was generated as described below. Accordingly, in order to predict an amount of blood loss volume at which a particular subject will collapse, the subject can be subjected to -15 mmHg LBNP for five minutes as a physical perturbation, and the resulting change in the subject's blood pressure waveform data (as measured, e.g., by a continuous noninvasive blood pressure monitor) can be analyzed against the generated model to provide an accurate predication of the blood loss volume at which the subject will suffer cardiovascular collapse. As an alternative to LBNP, an ITD can be used to provide the physical perturbation for generating the model and/or for making predictions based on the model.

As another exemplary use case, an ITD can be used to provide a physical perturbation to enhance estimates of blood loss. A model can be constructed by imposing, e.g., with an ITD, a passive negative pressure on the airway of a possibly bleeding subject. The change in the subject's blood pressure waveform before, during, and/or after the perturbation can be measured and correlated with an actual measured blood loss volume. Thereafter, a bleeding patient can be subjected to the same negative airway pressure (e.g., with an ITD) for one or more breaths, and data about the resulting change in the blood pressure waveform (e.g., measured using a noninvasive blood pressure monitor) can be analyzed against the model to provide an accurate prediction of the volume of blood lost by the patient.

As yet another exemplary use case, the techniques of certain embodiments can be used to estimate a subject's state of fatigue or sleepiness, which can be considered physiological states in accordance with such embodiments. Response time, type of response (motor, verbal, etc.) and/or accuracy of response to a physical perturbation are reflective of an individual's state of fatigue or sleepiness. Emerging models will incrementally account for an individual's initial state of fatigue or sleepiness. With each perturbation, improved estimates and predictions of cognitive fatigue, sleepiness and performance will become available. Moreover, such embodiments can enable the analytical computation of statistically based measures of reliability of the model predictions in the form of prediction intervals or windows. As the time interval increases in size, a greater number of perturbations can be analyzed and statistically more accurate estimations and predictions of physiological state and clinical condition(s) can be produced. The results may be used to predict individual-specific fatigue, sleepiness and performance impairment, which may help in planning improved sleep/wake schedules, as well as optimizing timing and dosing of fatigue countermeasures, in order to attain peak performance at desired times of the day

## Diagnostic and Therapeutic Techniques

Thus, using physical perturbations, various embodiments can be used to generate a model of a physiological state and, using that model, provide estimations and/or predictions regarding the physiological state, recommend therapeutic strategies, and/or implement recommended strategies.

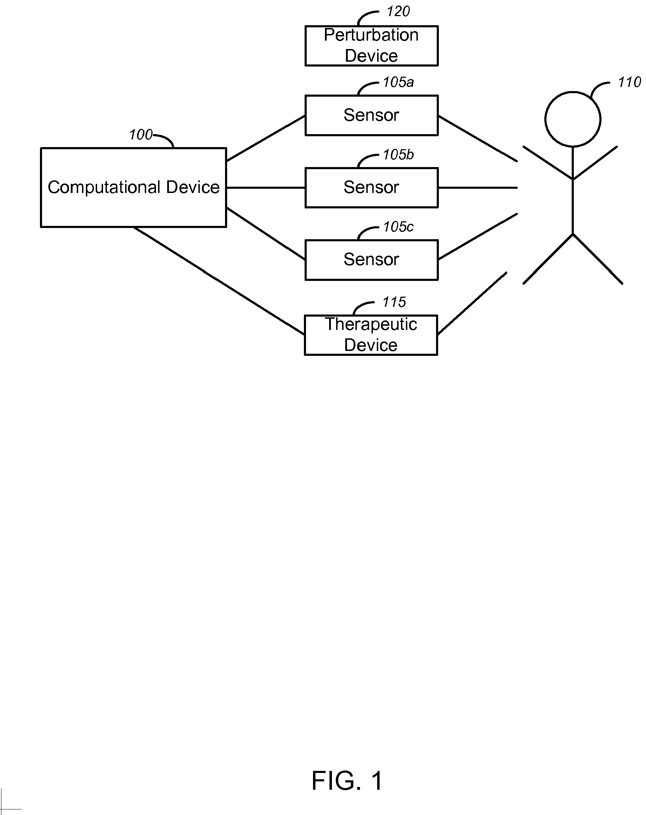

A general overview of a structure used in certain embodiments is provided by Fig. 1. The structure includes a computer system 100 in communication with one or more sensors 105, which are configured to obtain physiological data from the subject (e.g., animal or human test subject or patient) 110. An example of a computer system 100 that can be used in some embodiments is described in further detail below. In general, however, the computer system 100 can be any system of one or more computers that are capable of performing the techniques described herein. In a particular embodiment, for example, the computer system 100 is capable of reading values from the physiological sensors 105, generating models of physiological state from those sensors, and/or employing such models to make individual-specific estimations, predictions, or other diagnoses, displaying the results, recommending and/or implementing a therapeutic treatment as a result of the analysis, and/or archiving (learning) these results for use in future, model building and predictions

The sensors 105 can be any of a variety of sensors (including without limitation those described above) for obtaining physiological data from the subject. By way of example, in an embodiment one or more sensors 105 might obtain, e.g., using one or more of the techniques described above, continuous physiological waveform data, such as continuous blood pressure. Input from the sensors 105 can constitute continuous data signals and/or outcomes that can be used to generate, and/or can be applied to, a predictive model as described below.

In some cases, the structure might include a therapeutic device 115 (also referred to herein as a "physiological assistive device"), which can be controlled by the computer system 100 to administer therapeutic treatment, in accordance with the recommendations developed by analysis of a patient's physiological data. Examples of therapeutic devices can include a cardiac assist device, a ventilator, an automatic implantable cardioverter defibrillator ("AICD"), pacemakers, an extracorporeal membrane oxygenation circuit, a positive airway pressure ("PAP") device (including without limitation a continuous positive airway pressure ("cPAP") device or the like), an anesthesia machine, an integrated critical care system, a medical robot, intravenous and/or intra-arterial pumps that can provide fluids and/or therapeutic compounds (e.g., through intravenous injection), a heating/cooling blanket, and/or the like.

In a set of embodiments, the system of Fig. 1 further comprises a perturbation device 120. In some embodiments, the perturbation device 120 might be the same device as the therapeutic device 115, although this is not the case in all embodiments. The term "perturbation device," as used herein, means any device, whether operated manually or automatically, that can be used to produce a physical perturbation of the subject's body (or a portion thereof), in accordance with various embodiments. Examples include, but are not limited to, an LBNP chamber, ITD, blood pressure cuffs, and/or any other device that can exert a positive and/or negative pressure on a subject's body (or portion of a subject's body, such as the airway, extremities, etc.); a fluid and/or medication delivery system, such as an auto-infuser, intravenous pump or auto-infuser, etc., an electricity delivery system, such as an automatic implantable cardioverter defibrillator or a pacemaker, a heating/cooling blanket, and/or any other type of physical stimulant (e.g., an induced change in the subject's position, an audible sound, a change in temperature or lighting, etc.)

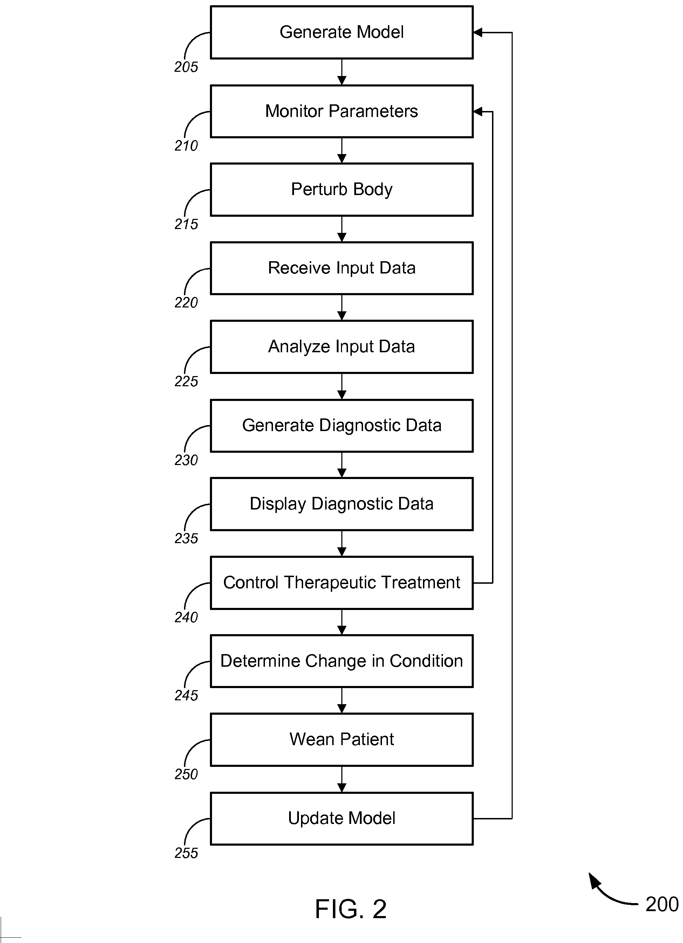

Figs. 2-4 illustrate methods in accordance with various embodiments. While the methods of Figs. 2-4 are illustrated, for ease of description, as different methods, it should be appreciated that the various techniques and procedures of these methods can be combined in any suitable fashion, and that, in some embodiments, the methods depicted by Figs. 2-4 can be considered interoperable and/or as portions of a single method. Similarly, while the techniques and procedures are depicted and/or described in a certain order for purposes of illustration, it should be appreciated that certain procedures may be reordered and/or omitted within the scope of various embodiments. Moreover, while the methods illustrated by Figs. 2-4 can be implemented by (and, in some cases, are described below with respect to) the computer system 100 of Fig. 1 (or components thereof), these methods may also be implemented using any suitable hardware implementation. Similarly, while the computer system 100 of Fig. 1 (and/or components thereof) can operate according to the methods illustrated by Figs. 2-4 (e.g., by executing instructions embodied on a computer readable medium), the system 100 can also operate according to other modes of operation and/or perform other suitable procedures.

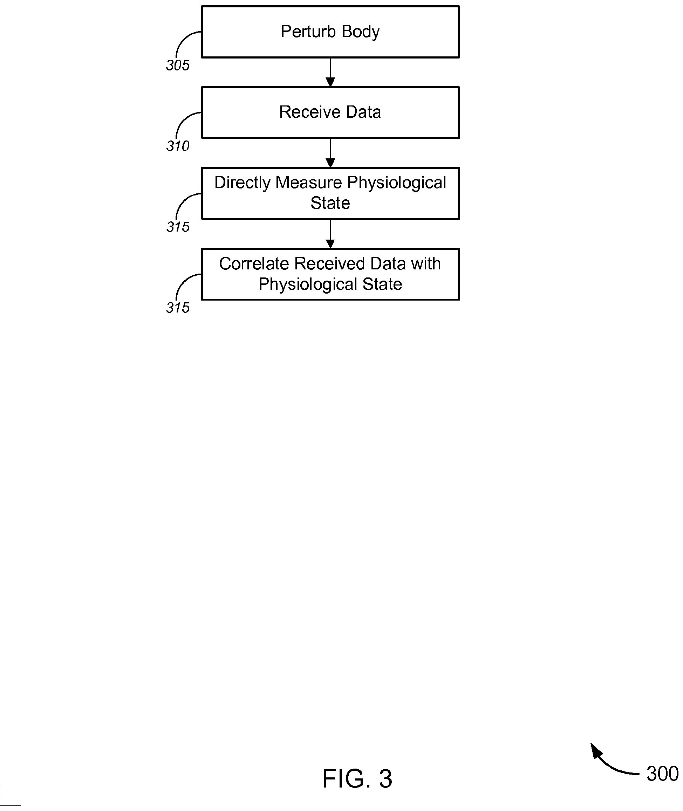

Fig. 2 illustrates a method 200 of enhanced medical monitoring, in accordance with a set of embodiments. The method 200 comprises, at block 205, generating a model of a physiological state, e.g., with a computer system. There are a variety of techniques for generating a model in accordance with different embodiments, some of which are described in further detail above. Another exemplary technique for generating a model of a physiological state is described below with respect to Fig. 3. Any suitable technique or model may be employed in accordance with various embodiments.

At block 210, the method 200 comprises monitoring one or more physiological parameters of a patient or other subject. As noted above, a variety of physical parameters can be monitored, invasively and/or non-invasively, depending on the nature of the anticipated physiological state of the patient. For example, if cardiovascular collapse is a concern, the patient's blood pressure and/or other parameters might be monitored. In an aspect, monitoring the one or more physical parameters might comprise receiving, e.g., from a physiological sensor, continuous waveform data, which can be sampled as necessary, including without limitation, before, during, and/or after a physical perturbation of the patient's body.

At block 215, the method comprises perturbing the patient's body, or a portion thereof. (As used herein, unless specifically indicated otherwise, physically perturbing any portion of a subject's body can be considered to be perturbing the patient's body itself.) In some cases, physically perturbing the patient's body might involve the use of a perturbation device, as described above, although this is not necessary. In some cases, the body might perturb itself through a change in position, a spontaneous breath, or a premature ventricular contraction or other cardiac dysrhythmia, to name a few examples. In such cases, perturbing the patient's body might simply comprise monitoring and/or detecting the self-perturbation of the body. Many different types of physical perturbations are described above, and any of these, or other modes of perturbation may be employed in accordance with the method 200.

At block 220, the method 200 comprises receiving (e.g., at a computer system), a set of input data. In most cases, as noted above, this input data is obtained by one or more physiological sensors, and the input data pertains to one or more physiological parameters of the patient (which can include, without limitation, some or all of the parameters described above), either before, during, and/or after perturbation of the patient's body. In an aspect, the sensors may be noninvasive sensors (although invasive sensors can be used as well), and might include, without limitation, electrocardiograph sensors, impedance cardiograph sensors, pulse oximeters, near infrared sensors, continuous noninvasive blood pressure sensors, transcranial Doppler sensors, and/or the like. Such sensors can obtain data about a variety of physiological conditions of the patient, including without limitation, continuous physiological waveform data, such as blood pressure waveform data. The sensors, then, can provide the received data to the computer system, e.g., through standard communication facilities, such as a cable, USB, Bluetooth, and/or the like.

At block 225, the input data is analyzed to determine a patient response to the one or more physical perturbations. This analysis might, comprise, for example, identifying a change in the monitored physical parameter based on data samples taken before the perturbation, during the perturbation and/or after the perturbation. In an aspect, the analysis might comprise analyzing the input data against a model of a specified physiological state. In some cases, the model is pre-existing (i.e., generated prior to receiving the input data), although, as noted above, the model can be refined using the input data and the results of the analysis itself. In an exemplary case, the model provides an algorithm to which the input data can be applied, to produce output data relating to an estimated/predicted physiological state of the patient that corresponds to the patient's measured physiological parameters.

Thus, the analysis of the input data against the model can be used to generate diagnostic data concerning the physiological state of the patient (block 230). The type of diagnostic data generated generally will depend on the nature of the monitored physiological parameter(s) and/or the anticipated physiological state(s) that the patient might possibly experience. In general, the diagnostic data might comprise an estimated current value of one or more physiological state of the patient, a predicted future value of the physiological state(s), and/or the like. In other cases, the diagnostic data might comprise a recommended treatment option for at least one of the physiological state(s) of the patient. Merely by way of example, the diagnostic data might include a recommendation to: optimize the hemodynamics of the patient; adjust a ventilator pressure, volume, etc.; adjust administration of intravenous fluids and/or medications; change patient position; or implement surgical therapy.

Alternatively and/or additionally, the diagnostic data might comprise data about a threshold condition (e.g., blood pressure value, volume of blood loss, etc.) at which the patient will suffer a particular physiological state (e.g., cardiovascular collapse). Merely by way of example, in some cases, the model might correlate certain physiological parameter values with the onset of a specified physiological state or condition, such as neurogenic or septic shock, hemodynamic decompensation, seizure, etc. The model, then, can be used to predict not only values of future physiological states and/or parameters, but also such value(s) that would likely result in the onset of a particular physiological state or condition, based on correlation between the received data about the physiological parameters and the predictions specified by the model.

At block 235, the method 200 comprises displaying (e.g., with a display device, such as a computer monitor, a device integrated with a monitoring system, etc.) at least a portion of the diagnostic data. Many different types of displays are possible, including without limitation, textual displays of data, graphical displays (e.g., using one or more graphically-rendered gauges, such as horizontal or vertical bars to illustrate a current estimated physiological state, a predicted physiological state, a prediction of what physiological parameters will be measured if and when the will patient experience the physiological state, etc.), and/or the like. In some cases, displaying the diagnostic data might comprise displaying a tracing of estimated and/or predicted values of the physiological state (and/or physiological parameters) over time, and/or a predicted physiological state and/or parameter value that might result in the onset of a condition, such as cardiovascular collapse.

At block 240, the method 200 comprises controlling a therapeutic treatment of the patient's physiological state, based at least in part on the diagnostic data. More specifically, in some cases the computer system has the ability to participate not only in the diagnosis of a physiological state, but also in the treatment of that condition, based (in some cases) upon the results of the system's analysis of the measured physiological parameters against the model. Controlling a therapeutic treatment can include many different operations, according to the functionality of the particular embodiment and/or the preferences of the operator.

Merely by way of example, controlling a therapeutic treatment might comprise implementing any recommended treatment options, including specifically controlling any therapeutic devices that are subject to computer control. Merely by way of example, as noted above, the system might comprise one or more therapeutic devices, such as intravenous pumps, ventilators, anesthesia machines, integrated critical care systems, medical robots, auto-infusers that can provide fluids and/or therapeutic compounds (e.g., through intravenous injection), and/or the like, and the system might control such a therapeutic device to administer the recommended course of treatment, either automatically and/or based on operator input (e.g., confirmation of a recommended treatment option, etc.).

In some cases, the operations of perturbing the body of the patient, receiving the input data, analyzing the input data, and generating the diagnostic data can be repeated, as illustrated by Fig. 2. Thus, for example, a patient can be monitored continuously, with new data being obtained by the sensors, provided to the computer, and applied to the model, through multiple iterations of the perturbation and/or after administration of the therapeutic treatment. The method 200, then, can comprise receiving a plurality of sets of input data, analyzing that data to produce multiple sets of diagnostic data, and providing an updated (e.g., continuously or periodically updated) display of the diagnostic data.

Analysis of these multiple sets of data can be used to determine a change in the physiological state of the patient (block 245), either as a result of the therapeutic treatment, as a function of the passage of time, and/or through other factors. As a result, new therapeutic options might be recommended. In particular, the method 200 might comprise, at block 250, weaning the patient from a physiological assistive device (e.g., a therapeutic device). Merely by way of example, if a ventilated patient begins to show signs of recovering from the physiological state that required mechanical ventilation (as determined, e.g., by continuing analysis of the patient's response to a physical perturbation as indicated by the measured physical parameters), the operation of the ventilator can be adjusted or stopped altogether, either automatically and/or through manipulation by the operator. Control of intravenous fluid/drug delivery by an intravenous pump, etc., can be implemented in similar fashion.

In other cases, the method 200 might comprise updating the model of the physiological state based, at least in part, on the input data and/or the analysis of that data (block 255). Merely by way of example, in some cases, collected data might be used to refine the model. In other cases, if the monitored parameters produce ambiguity when applied to the model, the system might collect data (e.g., from additional sensors) on additional parameters to resolve the ambiguities.

Fig. 3 illustrates a method of generating a model of a physiological state, which can be used, for example, to analyze input data as described with respect to Fig. 2. The method 300 comprises, at block 305, physically perturbing the body of one or more test subjects, for example, as described above. (As noted above, in some cases, perturbing the body might simply comprise monitoring or detecting a self-perturbation of the body.) At block 310, the method 300 further comprises receiving a plurality of physiological data sets (e.g., obtaining data, such as waveform data, from a plurality of physiological sensors monitoring the subject). The process of obtaining and/or receiving physiological data sets can be similar to that described above. At block 315, the method 300 comprises directly measuring the subject's physiological state with a reference sensor, to obtain a plurality of physiological state measurements. The nature of the reference sensor(s), and/or the measurements, often will depend on the physiological state that is being studied. For example, as noted above, the test subject may be instrumented as described above.

At block 315, the method 300 comprises correlating the received data with the measured physiological state of the test subject. It is often the case that the correlation between a specified physiological state and a given set of one or more physiological parameters is non-trivial. Merely by way of example, relationships between a physiological state and one or more physiological parameters may be complex and non-linear. Moreover, some combination of different physiological parameters (each having their own coefficient and/or polynomial order) might be most predictive of a current or future physiological state (such as hemodynamic decompensation) in various circumstances.

Accordingly, certain embodiments employ advanced computational techniques, such as those described in the '743 Publication, to identify this correlation. Merely by way of example, a particular embodiment employs a self-learning predictive model to identify correlations between a physiological state and other measured physiological parameters.

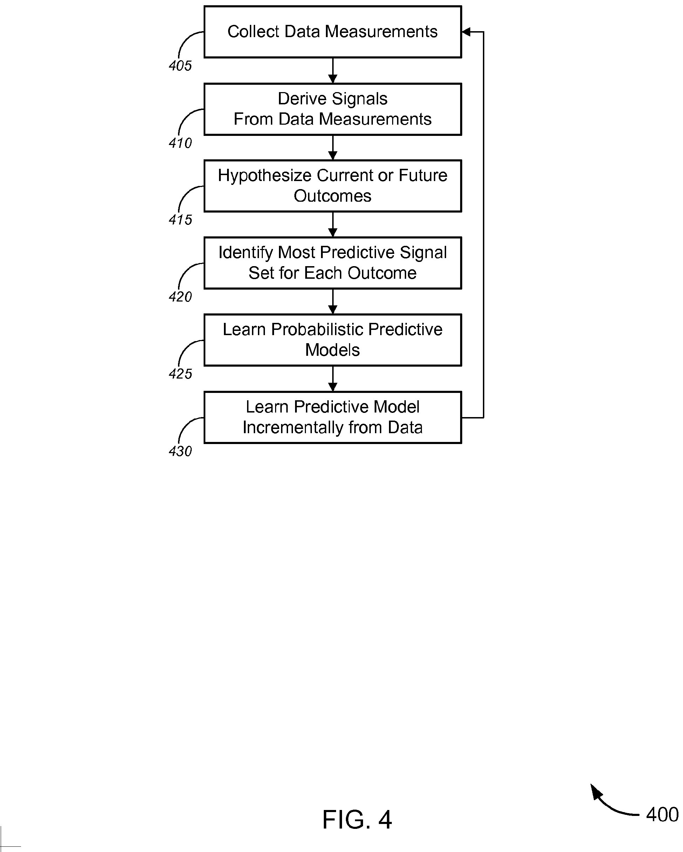

Fig. 4 illustrates a method of employing such a self-learning predictive model (or machine learning) method 400, according to some embodiments. In particular, the method 400 can be used to correlate physiological data received from a subject sensor with a measured physiological state. The method 400 begins at block 405 by collecting raw data measurements that may be used to derive a set of $D$ data signals $s_1, ..., s_D$ as indicated at block 410 (each of the data signals s being, in a particular case, input from one or many different physiological sensors). Embodiments are not constrained by the type of measurements that are made at block 405 and may generally operate on any data set. For example, data signals can be retrieved from a computer memory and/or can be provided from a sensor or other input device. A set of $K$ current or future outcomes $\vec{o} = (o_1, ..., o_K)$ is hypothesized at block 415 (the outcomes o being, in this case, past and/or future physiological states). The method autonomously generates a predictive model $M$ that relates the derived data signals $\vec{s}$ with the outcomes $\vec{o}$. As used herein, "autonomous," means "without human intervention."

As indicated at block 420, this is achieved by identifying the most predictive set of signals $S_k$, where $S_k$ contains at least some (and perhaps all) of the derived signals $s_1, ..., s_D$ for each outcome $o_k$, where $k \in \{1, ..., K\}$. A probabilistic predictive model $\hat{o}_k = M_k(S_k)$ is learned at block 425, where $\hat{o}_k$ is the prediction of outcome $o_k$ derived from the model

$M_k$ that uses as inputs values obtained from the set of signals $S_k$, for all $k \in \{1, ..., K\}$. The method 400 can learn the predictive models $\hat{o}_k = M_k(S_k)$ incrementally (block 430) from data that contains example values of signals $s_1, ..., s_D$ and the corresponding outcomes $o_1, ..., o_K$. As the data become available, the method 400 loops so that the data are added incrementally to the model for the same or different sets of signals $S_k$, for all $k \in \{1, ... , K\}$.

While the description above outlines the general characteristics of the methods, additional features are noted. A linear model framework may be used to identify predictive variables for each new increment of data. In a specific embodiment, given a finite set of data of signals and outcomes $\{(\vec{s}_1, \vec{o}_1), (\vec{s}_2, \vec{o}_2), ... \}$, a linear model may be constructed that has the form, for all $k \in \{1, ..., K\}$,

$$\hat{o}_k = f_k\left(a_0 + \sum_{i=1}^{d} a_i s_i\right) \qquad \text{(Eq. 11)}$$

where $f_k$ is any mapping from one input to one output, and $a_0, a_1, ..., a_d$ are the linear model coefficients. The framework used to derive the linear model coefficients may estimate which signals $s, s_1, ..., s_d$ are not predictive and accordingly sets the corresponding coefficients $a_0, a_1, ..., a_d$ to zero. Using only the predictive variables, the model builds a predictive density model of the data, $\{(\vec{s}_1, \vec{o}_1), (\vec{s}_2, \vec{o}_2), ... \}$. For each new increment of data, a new predictive density models can be constructed.

In some embodiments, a prediction system can be implemented that can predict future results from previously analyzed data using a predictive model and/or modify the predictive model when data does not fit the predictive model. In some embodiments, the prediction system can make predictions and/or to adapt the predictive model in real-time. Moreover, in some embodiments, a prediction system can use large data sets not only to create the predictive model, but also predict future results as well as adapt the predictive model.

In some embodiments, a self-learning, prediction device can include a data input, a processor and an output. Memory can include application software that when executed can direct the processor to make a prediction from input data based on a predictive model. Any type of predictive model can be used that operates on any type of data. In some embodiments, the predictive model can be implemented for a specific type of data. In some embodiments, when data is received the predictive model can determine whether it understands the data according to the predictive model. If the data is understood, a prediction is made and the appropriate output provided based on the predictive model. If the data is not understood when received, then the data can be added to the predictive model to modify the model. In some embodiments, the device can wait to determine the result of the specified data and can then modify the predictive model accordingly. In some embodiments, if the data is understood by the predictive model and the output generated using the predictive model is not accurate, then the data and the outcome can be used to modify the predictive model. In some embodiments, modification of the predictive model can occur in real-time.

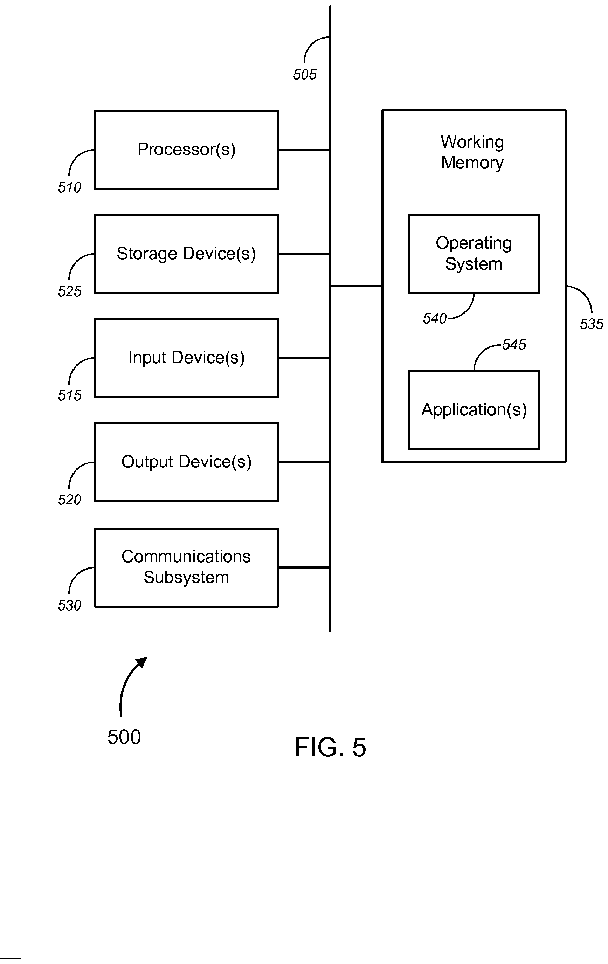

Fig. 5 provides a schematic illustration of one embodiment of a computer system 500 that can perform the methods provided by various other embodiments, as described herein, and/or can function as the computer system of Fig. 1, a patient monitoring system, and/or the like. It should be noted that Fig. 5 is meant only to provide a generalized illustration of various components, of which one or more (or none) of each may be utilized as appropriate. Fig. 5, therefore, broadly illustrates how individual system elements may be implemented in a relatively separated or relatively more integrated manner.

The computer system 500 is shown comprising hardware elements that can be electrically coupled via a bus 505 (or may otherwise be in communication, as appropriate). The hardware elements may include one or more processors 510, including without limitation one or more general-purpose processors and/or one or more special-purpose processors (such as digital signal processing chips, graphics acceleration processors, and/or the like); one or more input devices 515, which can include without limitation a mouse, a keyboard and/or the like; and one or more output devices 520, which can include without limitation a display device, a printer and/or the like.

The computer system 500 may further include (and/or be in communication with) one or more storage devices 525, which can comprise, without limitation, local and/or network accessible storage, and/or can include, without limitation, a disk drive, a drive array, an optical storage device, solid-state storage device such as a random access memory ("RAM") and/or a read-only memory ("ROM"), which can be programmable, flash-updateable and/or the like. Such storage devices may be configured to implement any appropriate data stores, including without limitation, various file systems, database structures, and/or the like.

The computer system 500 might also include a communications subsystem 530, which can include without limitation a modem, a network card (wireless or wired), an infra-red communication device, a wireless communication device and/or chipset (such as a Bluetooth™ device, an 802.11 device, a WiFi device, a WiMax device, a WWAN device, cellular communication facilities, etc.), and/or the like. The communications subsystem 530 may permit data to be exchanged with a network (such as the network described below, to name one example), with other computer systems, and/or with any other devices described herein. For example, in various embodiments, the communications subsystem provides connectivity for the computer to control and/or receive data from one or more sensors, and/or to control a therapeutic device, as described above. Alternatively and/or additionally, the sensors and/or the therapeutic device might be integrated with the computer system itself.

In many embodiments, the computer system 500 will further comprise a working memory 535, which can include a RAM or ROM device, as described above. The computer system 500 also may comprise software elements, shown as being currently located within the working memory 535, including an operating system 540, device drivers, executable libraries, and/or other code, such as one or more application programs 545, which may comprise computer programs provided by various embodiments, and/or may be designed to implement methods, and/or configure systems, provided by other embodiments, as described herein. Merely by way of example, one or more procedures described with respect to the method(s) discussed above might be implemented as code and/or instructions executable by a computer (and/or a processor within a computer); in an aspect, then, such code and/or instructions can be used to configure and/or adapt a general purpose computer (or other device) to perform one or more operations in accordance with the described methods.

A set of these instructions and/or code might be encoded and/or stored on a non-transitory computer readable storage medium, such as the storage device(s) 525 described above. In some cases, the storage medium might be incorporated within a computer system, such as the system 500. In other embodiments, the storage medium might be separate from a computer system (i.e., a removable medium, such as a compact disc, micro- or thumb-drive etc.), and/or provided in an installation package, such that the storage medium can be used to program, configure and/or adapt a general purpose computer with the instructions/code stored thereon. These instructions might take the form of executable code, which is executable by the computer system 500 and/or might take the form of source and/or installable code, which, upon compilation and/or installation on the computer system 500 (e.g., using any of a variety of generally available compilers, installation programs, compression/decompression utilities, etc.) then takes the form of executable code.

It will be apparent to those skilled in the art that substantial variations may be made in accordance with specific requirements. For example, customized hardware (such as programmable logic controllers, field-programmable gate arrays, application-specific integrated circuits, and/or the like) might also be used, and/or particular elements might be implemented in hardware, software (including portable software, such as applets, etc.), or both. Further, connection to other computing devices such as network input/output devices may be employed.

As mentioned above, in one aspect, some embodiments may employ a computer system (such as the computer system 500) to perform methods in accordance with various embodiments of the invention. According to a set of embodiments, some or all of the procedures of such methods are performed by the computer system 500 in response to processor 510 executing one or more sequences of one or more instructions (which might be incorporated into the operating system 540 and/or other code, such as an application program 545) contained in the working memory 535. Such instructions may be read into the working memory 535 from another computer readable medium, such as one or more of the storage device(s) 525. Merely by way of example, execution of the sequences of instructions contained in the working memory 535 might cause the processor(s) 510 to perform one or more procedures of the methods described herein.

The terms "machine readable medium" and "computer readable medium," as used herein, refer to any medium that participates in providing data that causes a machine to operate in a specific fashion. In an embodiment implemented using the computer system 500, various computer readable media might be involved in providing instructions/code to processor(s) 510 for execution and/or might be used to store and/or carry such instructions/code (e.g., as signals). In many implementations, a computer readable medium is a non-transitory, physical and/or tangible storage medium. Such a medium may take many forms, including but not limited to, non-volatile media, volatile media, and transmission media. Non-volatile media includes, for example, optical and/or magnetic disks, such as the storage device(s) 525. Volatile media includes, without limitation, dynamic memory, such as the working memory 535. Transmission media includes, without limitation, coaxial cables, copper wire and fiber optics, including the wires that comprise the bus 505, as well as the various components of the communication subsystem 530 (and/or the media by which the communications subsystem 530 provides communication with other devices). Hence, transmission media can also take the form of waves (including without limitation radio, acoustic and/or light waves, such as those generated during radio-wave and infra-red data communications).

Common forms of physical and/or tangible computer readable media include, for example, a floppy disk, a flexible disk, a hard disk, magnetic tape, or any other magnetic medium, a CD-ROM, any other optical medium, punch cards, paper tape, any other physical medium with patterns of holes, a RAM, a PROM, and EPROM, a FLASH-EPROM, any other memory chip or cartridge, a carrier wave as described hereinafter, or any other medium from which a computer can read instructions and/or code.

Various forms of computer readable media may be involved in carrying one or more sequences of one or more instructions to the processor(s) 510 for execution. Merely by way of example, the instructions may initially be carried on a magnetic disk and/or optical disc of a remote computer. A remote computer might load the instructions into its dynamic memory and send the instructions as signals over a transmission medium to be received and/or executed by the computer system 500. These signals, which might be in the form of electromagnetic signals, acoustic signals, optical signals and/or the like, are all examples of carrier waves on which instructions can be encoded, in accordance with various embodiments of the invention.

The communications subsystem 530 (and/or components thereof) generally will receive the signals, and the bus 505 then might carry the signals (and/or the data, instructions, etc. carried by the signals) to the working memory 535, from which the processor(s) 505 retrieves and executes the instructions. The instructions received by the working memory 535 may optionally be stored on a storage device 525 either before or after execution by the processor(s) 510.

While certain features and aspects have been described with respect to exemplary embodiments, one skilled in the art will recognize that numerous modifications are possible. For example, the methods and processes described herein may be implemented using hardware components, software components, and/or any combination thereof. Further, while various methods and processes described herein may be described with respect to particular structural and/or functional components for ease of description, methods provided by various embodiments are not limited to any particular structural and/or functional architecture but instead can be implemented on any suitable hardware, firmware and/or software configuration. Similarly, while certain functionality is ascribed to certain system components, unless the context dictates otherwise, this functionality can be distributed among various other system components in accordance with the several embodiments.

Moreover, while the procedures of the methods and processes described herein are described in a particular order for ease of description, unless the context dictates otherwise, various procedures may be reordered, added, and/or omitted in accordance with various embodiments. Moreover, the procedures described with respect to one method or process may be incorporated within other described methods or processes; likewise, system components described according to a particular structural architecture and/or with respect to one system may be organized in alternative structural architectures and/or incorporated within other described systems. Hence, while various embodiments are described with-or without-certain features for ease of description and to illustrate exemplary aspects of those embodiments, the various components and/or features described herein with respect to a particular embodiment can be substituted, added and/or subtracted from among other described embodiments, unless the context dictates otherwise. Consequently, although several exemplary embodiments are described above, it will be appreciated that the invention is intended to cover all modifications and equivalents within the scope of the following claims.

## Claims

1. A method (200), comprising:

   physically perturbing (215) a body of a patient (110) with one or more physical perturbations;
   receiving (200), at a computer system (100; 500), a set of input data from one or more physiological sensors (105), the set of input data pertaining to one or more physiological parameters of the physically perturbed body of the patient (110);
   analyzing (225), with the computer system (100; 500), the input data to determine a patient (110) response to the one or more physical perturbations;
   generating diagnostic data, with the computer system (100; 500), concerning one or more physiological states of the patient; and
   displaying (235), with a display device, at least a portion of the diagnostic data;

   **characterized in that** analyzing the input data comprises: analyzing the input data against a pre-existing model; the method further comprising: generating (300) the pre-existing model, wherein generating (300) the pre-existing model comprises:

   physically perturbing (305) a test subject with the one or more physical perturbations;
   receiving data (310) pertaining to a plurality of more physiological parameters of a test subject to obtain a plurality of physiological data sets;
   directly measuring (315) the one or more physiological states of the test subject with a reference sensor to obtain a plurality of physiological state measurements; and
   correlating (315) the received data with the physiological state measurements of the test subject.

2. The method of claim 1, wherein physically perturbing (215) the body of the patient (110) comprises applying a lower body negative pressure to the body of the patient.

3. The method of claim 1, wherein physically perturbing (215) the body of the patient (110) comprises inducing a negative pressure on an airway of the patient during breathing or ventilation,

4. The method of claim 3, wherein the negative pressure is applied with an impedance threshold device.

5. The method of claim 3, wherein the negative pressure is applied passively.

6. The method of claim 1, wherein physically perturbing (215) the body of the patient (110) comprises detecting a self-perturbation of the body.

7. The method of claim 1, wherein physically perturbing (215) the body of the patient (110) comprises inducing a positive pressure on at least a portion of the body of the patient.

8. The method of claim 7, wherein the positive pressure is induced with a blood pressure cuff.

9. The method of claim 7, wherein inducing a positive pressure on at least a portion of the body comprises inducing a positive pressure on an airway of the patient during breathing or ventilation.

10. The method of claim 1, wherein physically perturbing (215) the body comprises delivering one or more medications to the patient.

11. The method of claim 1, wherein physically perturbing (215) the body comprises performing a valsalva maneuver.

12. The method of claim 1, wherein physically perturbing (215) the body comprises delivering a fluid to the patient.

13. The method of claim 1, wherein physically perturbing (215) the body comprises delivering electrical current to the patient.

14. The method of claim 1, wherein the diagnostic data comprises:

> - an estimated current value of at least one of the one or more physiological states of the patient, or
> - predicted future value of at least one of the one or more physiological states of the patient, or
> - a predicted future value of at least one of the one or more physiological states of the patient, or
> - a recommended treatment option for at least one of the one or more physiological states of the patient.

15. The method of claim 1, further comprising:

> producing a plurality of sets of diagnostic data by repeating the operations of physically perturbing the body of the patient, receiving a set of input data, analyzing the input data, and generating diagnostic data;
> determining a change in a physiological state of the patient, based at least in part on the plurality of sets of diagnostic data; and
> weaning the patient from a physiological assistive device, based on the determined change in the physiological condition of the patient.

16. The method of claim 15, wherein the physiological assistive device is selected from the group consisting of an intraortic balloon pump, a cardiac assist device, an automatic implantable cardioverter defibrillator, a ventilator, an extracorporeal membrane oxygenation device, a positive airway pressure device, an anesthesia machine, an integrated critical care system, a medical robot, an intravenous or intra-arterial pump, a heating blanket, and a cooling blanket.

17. The method of claim 1, wherein at least one of the one or more sensors (105) is noninvasive.

18. The method of claim 1, wherein at least one of the one or more sensors (105) is selected from the group consisting of a blood pressure sensor, an intracranial pressure monitor, a central venous pressure monitoring catheter, an electroencephalograph, a cardiac monitor, a transcranial Doppler sensor, a transthoracic impedance plethysmograph, a pulse oximeter, a near infrared spectrometer, a ventilator, an accelerometer, an electrooculogram, a transcutaneous glucometer, an electrolyte sensor, and an electronic stethoscope.

**19.** The method of claim 1, wherein the input data comprises blood pressure waveform data, or plethysmograph waveform data.

**20.** The method according to any of the preceding claims, wherein the pre-existing model provides an algorithm to which the input data are applied, to produce output data relating to an estimated/predicted physiological state of the patient.

**21.** The method according to any of the preceding claims, wherein the pre-existing model is a self-learning predictive model to identify correlations between a physiological state and other measured physiological parameters.

**22.** The method according to claim 21, wherein the method autonomously generates the predictive model.

**23.** The method according to any of the preceding claims, wherein correlating the received data with the physiological state measurements of the test subject comprises:

identifying a most-predictive set of signals $S_k$ out of a set of signals $s_1, s_2, ..., s_D$ for each of one or more outcomes $o_k$, wherein the most-predictive set of signals $S_k$ corresponds to a first data set representing a first physiological parameter, and wherein each of the one or more outcomes $o_k$ represents a physiological state measurement; autonomously learning a set of probabilistic predictive models $\hat{o}_k = M_k(S_k)$, where $\hat{o}_k$ is a prediction of outcome $o_k$ derived from a model $M_k$ that uses as inputs values obtained from the set of signals $S_k$; and repeating the operation of autonomously learning incrementally from data that contains examples of values of signals $s_1, s_2, ..., s_D$ and corresponding outcomes $o_1, o_2, ..., o_K$.

**24.** An apparatus, comprising:
a computer readable medium having encoded thereon a set of instructions executable by one or more computers to perform one or more operations, the set of instructions comprising:

instructions for receiving, at a computer system (100; 500), a set of input data from one or more physiological sensors (105), the set of input data pertaining to one or more physiological parameters of a physically perturbed body of a patient;
**characterized by** instructions for analyzing, with the computer system (100; 500), the input data against a pre-existing model to determine a patient response to the one or more physical perturbations;
instructions for generating the pre-existing model, wherein generating the pre-existing model comprises:

physically perturbing a test subject with the one or more physical perturbations;
receiving data pertaining to a plurality of more physiological parameters of a test subject to obtain a plurality of physiological data sets;
directly measuring the one or more physiological states of the test subject with a reference sensor to obtain a plurality of physiological state measurements; and
correlating the received data with the physiological state measurements of the test subject;

instructions for generating diagnostic data concerning one or more physiological states of the patient; and
instructions for displaying, with a display device, at least a portion of the diagnostic data.

**25.** The apparatus according to claim 24, further comprising:

one or more processors (510);
the computer readable medium in communication with the one or more processors, the computer readable medium having encoded thereon the set of instructions executable by the computer system (100; 500) to perform the one or more operations.

**26.** The apparatus of claim 25, further comprising:
a perturbation device (120) configured to physically perturb the body of the patient with one or more physical perturbations.

**27.** A method according to claim 1,
wherein the step of physical perturbing (215) the body of the patient (110) comprises applying lower body negative pressure to the subject for an amount of time sufficient to cause variation in a blood pressure of the subject but insufficient to provoke cardiovascular collapse in the subject;

monitoring continuous blood pressure waveform data of the subject using a non- invasive monitor, including recording a plurality of blood pressure readings of the subject, the plurality of blood pressure readings comprising a first set of one or more blood pressure readings prior to application of the lower body negative pressure and a second set of one or more blood pressure readings during or after application of the lower body negative pressure;

the step of analyzing (225) comprises analyzing, with a computer system (100; 500), the blood pressure waveform data against a pre-exiting model;

the step of generating diagnostic data comprises generating, based on analysis of the blood pressure waveform data, an estimated volume of blood loss or lower body negative pressure at which the subject will suffer cardiovascular collapse; and

the step of displaying comprises displaying information about the volume of blood loss at which the subject will suffer cardiovascular collapse.

**Patentansprüche**

1. Verfahren (200), umfassend:

   physikalisches Stören (215) eines Körpers eines Patienten (110) mit einer oder mehr physikalischen Störungen;
   Empfangen (200), auf einem Computersystem (100; 500), eines Satzes von Eingangsdaten von einem oder mehreren physiologischen Sensoren (105), der Satz von Eingangsdaten gehörend zu einem oder mehreren physiologischen Parametern des physikalisch gestörten Körpers des Patienten (110);
   Analysieren (225), mit dem Computersystem (100; 500), der Eingangsdaten, um eine Reaktion des Patienten (110) auf eine oder mehrere physikalische Störungen zu bestimmen;
   Generieren von Diagnosedaten mit dem Computersystem (100, 500) betreffend einen oder mehr physiologische Zustände des Patienten; und
   Anzeigen (235), mit einer Anzeigevorrichtung, von mindestens einem Teil der Diagnosedaten;
   **dadurch gekennzeichnet, dass** das Analysieren der Eingangsdaten das Analysieren der Eingangsdaten gegen ein vorher existierendes Modell umfasst;
   das Verfahren ferner umfassend das Generieren (300) des vorher existierenden Modells, wobei das Generieren (300) des vorher existierenden Modells Folgendes umfasst:

   physikalisches Stören (305) eines Versuchssubjekts mit der einen oder den mehreren physikalischen Störungen;
   Empfangen von Daten (310), gehörend zu einer Vielzahl von mehr physiologischen Parametern eines Versuchssubjekts, um eine Vielzahl physiologischer Datensätze zu erhalten;
   direktes Messen (315) des einen oder der mehreren physiologischen Zustände des Versuchssubjekts mit einem Referenzsensor, um eine Vielzahl physiologischer Zustandsmessungen zu erhalten; und
   Korrelieren (315) der empfangenen Daten mit den physiologischen Zustandsmessungen des Versuchssubjekts.

2. Verfahren nach Anspruch 1, wobei das physikalische Stören (215) des Körpers des Patienten (110) das Anwenden eines niedrigen negativen Körperdrucks auf den Körper des Patienten umfasst.

3. Verfahren nach Anspruch 1, wobei das physikalische Stören (215) des Körpers des Patienten (110) das Herbeiführen eines negativen Drucks auf einen Atemweg des Patienten während der Atmung oder Ventilation umfasst.

4. Verfahren nach Anspruch 3, wobei der negative Druck mit einer Impedanzschwellenwertvorrichtung angewendet wird.

5. Verfahren nach Anspruch 3, wobei der negative Druck passiv angewendet wird.

6. Verfahren nach Anspruch 1, wobei das physikalische Stören (215) des Körpers des Patienten (110) das Erkennen einer Selbststörung des Körpers umfasst.

7. Verfahren nach Anspruch 1, wobei die physikalische Störung (215) des Körpers des Patienten (110) das Herbeiführen eines positiven Drucks auf mindestens einem Teil des Körpers des Patienten umfasst.

8. Verfahren nach Anspruch 7, wobei der positive Druck mit einer Blutdruckmanschette herbeigeführt wird.

9. Verfahren nach Anspruch 7, wobei das Herbeiführen eines positiven Drucks auf mindestens einem Teil des Körpers das Herbeiführen eines positiven Drucks auf einen Luftweg des Patienten während der Atmung oder Ventilation umfasst.

10. Verfahren nach Anspruch 1, wobei das physikalische Stören (215) des Körpers das Verabreichen von einer oder mehr Medikationen an den Patienten umfasst.

11. Verfahren nach Anspruch 1, wobei das physikalische Stören (215) des Körpers die Durchführung eines Valsalva-Manövers umfasst.

12. Verfahren nach Anspruch 1, wobei das physikalische Stören (215) des Körpers die Verabreichung einer Flüssigkeit an den Patienten umfasst.

13. Verfahren nach Anspruch 1, wobei die physikalische Störung (215) des Körpers die Verabreichung von elektrischem Strom an den Patienten umfasst.

14. Verfahren nach Anspruch 1, wobei die Diagnosedaten Folgendes umfassen:

- einen geschätzten aktuellen Wert von mindestens einem des einen oder der mehreren physiologischen Zustände des Patienten, oder
- einen vorhergesehenen zukünftigen Wert von mindestens einem des einen oder der mehreren physiologischen Zustände des Patienten, oder
- einen vorhergesehenen zukünftigen Wert von mindestens einem des einen oder der mehreren physiologischen Zustände des Patienten, oder
- eine empfohlene Behandlungsoption für mindestens einen des einen oder der mehreren physiologischen Zustände des Patienten.

15. Verfahren nach Anspruch 1, ferner umfassend:

das Erzeugen einer Vielzahl von Sätzen von Diagnosedaten durch Wiederholung der Vorgänge des physikalischen Störens des Körpers des Patienten, das Empfangen eines Satzes von Eingangsdaten, das Analysieren der Eingangsdaten und das Generieren von Diagnosearten;
das Bestimmen einer Veränderung in einem physiologischen Zustand des Patienten, basierend mindestens teilweise auf der Vielzahl von Sätzen von Diagnosedaten; und
das Absetzen des Patienten von einer physiologischen unterstützenden Vorrichtung, basierend auf der bestimmten Veränderung in dem physiologischen Zustand des Patienten.

16. Verfahren nach Anspruch 15, wobei die physiologische unterstützende Vorrichtung ausgewählt ist aus der Gruppe, bestehend aus einer intraortalen Ballonpumpe, einer kardialen Hilfsvorrichtung, einem automatisch implantierbaren Kardioverter-Defibrillator, einem Ventilator, einer extrakorporalen Membranoxygenationsvorrichtung, einer positiven Luftwegdruckvorrichtung, einer Anästhesiemaschine, einem integrierten Intensivpflegesystem, einem medizinischen Roboter, einer intravenösen oder intraarteriellen Pumpe, einer Heizdecke und einer Kühldecke.

17. Verfahren nach Anspruch 1, wobei mindestens einer des einen oder der mehreren Sensoren (105) nichtinvasiv ist.

18. Verfahren nach Anspruch 1, wobei mindestens einer des einen oder der mehreren Sensoren (105) ausgewählt ist aus der Gruppe, bestehend aus einem Blutdrucksensor, einem intrakranialen Druckmonitor, einem zentralen Venendrucküberwachungskatheter, einem Elektroenzephalografen, einem Herzmonitor, einem transkranialen Doppler-Sensor, einem transthorakalen Impedanzplethysmografen, einem Pulsoximeter, einem Nahinfrarot-Spektrometer, einem Ventilator, einem Akzelerometer, einem Elektrooculogramm, einem transkutanen Glukometer, einem Elektrolytsensor und einem elektronischen Stethoskop.

19. Verfahren nach Anspruch 1, wobei die Eingangsdaten Blutdruck-Wellenformdaten oder Plethysmograf-Wellenformdaten umfassen.

20. Verfahren nach einem der vorhergehenden Ansprüche, wobei das vorher existierende Modell einen Algorithmus bereitstellt, auf den die Eingangsdaten angewendet werden, um Ausgangsdaten betreffend einen geschätzten/vorhergesehenen physiologischen Zustand des Patienten zu erzeugen.

**21.** Verfahren nach einem der vorhergehenden Ansprüche, wobei das vorher existierende Modell ein selbstlernendes prädiktives Modell ist, um Korrelationen zwischen einem physiologischen Zustand und anderen gemessenen physiologischen Parametern zu identifizieren.

**22.** Verfahren nach Anspruch 21, wobei das Verfahren das prädiktive Modell autonom erzeugt.

**23.** Verfahren nach einem der vorhergehenden Ansprüche, wobei das Korrelieren der empfangenen Daten mit den physiologischen Zustandsmessungen der Versuchsperson Folgendes umfasst:

Identifizieren eines am meisten prädiktiven Satzes von Signalen $S_k$ aus einem Satz von Signalen $s_1, s_2 ... s_D$ für jedes von einem oder mehr Ergebnissen $o_k$, wobei der am meisten prädiktive Satz von Signalen $S_k$ einem ersten Datensatz, der einen ersten physiologischen Parameter darstellt, entspricht, und wobei jedes des einen oder der mehreren Ergebnisse $o_k$ eine physiologische Zustandsmessung darstellt;
autonomes Lernen eines Satzes von wahrscheinlichkeitstheoretischen prädiktiven Modellen $ô_k = M_k(S_k)$, wobei $ô_k$ eine Vorhersage eines Ergebnisses $o_k$ ist, abgeleitet von einem Modell $M_k$, das Eingaben Werte, erhalten von dem Satz von Signalen $Sk$ verwendet; und
Wiederholen des Vorgangs des autonomen Lernens inkremental von Daten, die Beispiele von Werten von Signalen $s_1, s_2 ... s_D$ enthalten und Ergebnissen $o_1, o_2 ..., o_k$ entsprechen.

**24.** Vorrichtung, umfassend:
ein computerlesbares Medium mit darauf codiert einem Satz von Befehlen, ausführbar durch einen oder mehrere Computer zur Durchführung von einem oder mehreren Operationen, der Satz von Befehlen umfassend:

Befehle zum Empfangen, auf einem Computersystem (100, 500), eines Satzes von Eingangsdaten von einem oder mehreren physiologischen Sensoren (105), der Satz von Eingangsdaten gehörend zu einem oder mehreren physiologischen Parametern eines physikalisch gestörten Körper eines Patienten;
**gekennzeichnet durch** Befehle zum Analysieren, mit dem Computersystem (100; 500), der Eingangsdaten gegen ein vorher existierendes Modell, um eine Patientenreaktion auf die eine oder mehreren physikalischen Störungen zu bestimmen;
Befehle zum Generieren des vorher existierenden Modells, wobei das Generieren des vorher existierenden Modells Folgendes umfasst:

physikalisches Stören eines Versuchssubjekts mit der einen oder den mehreren physikalischen Störungen;
Empfangen von Daten, gehörend zu einer Vielzahl von mehreren physiologischen Parametern eines Versuchssubjekts, um eine Vielzahl physiologischer Datensätze zu erhalten;
direktes Messen des einen oder der mehreren physiologischen Zustände des Versuchssubjekts mit einem Referenzsensor, um eine Vielzahl physiologischer Zustandsmessungen zu erhalten; und
Korrelieren der empfangenen Daten mit den physiologischen Zustandsmessungen des Versuchssubjekts;

Befehle zum Generieren von Diagnosedaten betreffend einen oder mehrere physiologische Zustände des Patienten; und
Befehle zum Anzeigen, mit einer Anzeigevorrichtung, von mindestens einem Teil der Diagnosedaten.

**25.** Vorrichtung nach Anspruch 24, ferner umfassend:

einen oder mehrere Prozessoren (510);
das computerlesbare Medium in Kommunikation mit dem einen oder den mehreren Prozessoren, das computerlesbare Medium mit darauf codiert den Satz von Befehlen, ausführbar von dem Computersystem (100; 500) zur Durchführung der einen oder mehrere Operationen.

**26.** Vorrichtung nach Anspruch 25, ferner umfassend:
eine Störungsvorrichtung (120), konfiguriert, um physikalisch den Körper des Patienten mit einem oder mehreren physikalischen Störungen zu stören.

**27.** Verfahren nach Anspruch 1,
wobei der Schritt des physikalischen Störens (215) des Körpers des Patienten (110) das Anwenden von niedrigem negativem Körperdruck auf das Subjekt während einer ausreichend langen Zeitdauer umfasst, ausreichend um Abweichung in einem Blutdruck des Subjekts zu verursachen, aber unzureichend, um kardiovaskulären Kollaps bei dem Subjekt zu verursachen;

das Überwachen von kontinuierlichen Blutdruck-Wellenformdaten des Subjekts unter Verwendung eines nichtinvasiven Monitors, einschließlich Aufzeichnung einer Vielzahl von Blutdruckablesungen des Subjekts, die Vielzahl von Blutdruckablesungen umfassend einen ersten Satz von einer oder mehr Blutdruckablesungen vor der Anwendung des niedrigen negativen Körperdrucks und eines zweiten Satzes von einer oder mehr Blutdruckablesungen während oder nach der Anwendung des niedrigen negativen Körperdrucks;

wobei der Schritt des Analysierens (225) das Analysieren, mit einem Computersystem (100; 500) der Blutdruck-Wellenformdaten gegen ein vorher existierendes Modell umfasst;

der Schritt des Generierens von Diagnosedaten das Generieren, basierend auf Analyse der Blutdruck-Wellenformdaten, eines geschätzten Volumens von Blutverlust oder niedrigem negativem Körperdruck, bei dem das Subjekt kardiovaskulären Kollaps erleidet; umfasst und

der Schritt des Anzeigens die Anzeige von Information über das Volumen von Blutverlust, bei dem das Subjekt kardiovaskulären Kollaps erleidet, umfasst.

## Revendications

1. Procédé (200) comprenant les étapes ci-dessous consistant à :

    perturber physiquement (215) le corps d'un patient (110) au moyen d'une ou plusieurs perturbations physiques ;
    recevoir (200), au niveau d'un système informatique (100 ; 500), un ensemble de données d'entrée en provenance d'un ou plusieurs capteurs physiologiques (105), l'ensemble de données d'entrée se rapportant à un ou plusieurs paramètres physiologiques du corps physiquement perturbé du patient (110) ;
    analyser (225), avec le système informatique (100 ; 500), les données d'entrée, en vue de déterminer la réponse d'un patient (110) à ladite une ou auxdites plusieurs perturbations physiques ;
    générer des données de diagnostic, avec le système informatique (100 ; 500), concernant un ou plusieurs états physiologiques du patient ; et
    afficher (235), avec un dispositif d'affichage, au moins une partie des données de diagnostic ;

    **caractérisé en ce que** l'étape d'analyse des données d'entrée consiste à : analyser les données d'entrée par rapport à un modèle préexistant ;
    le procédé comprenant en outre l'étape consistant à : générer (300) le modèle préexistant, dans lequel l'étape de génération (300) du modèle préexistant comprend les étapes ci-dessous consistant à :

    perturber physiquement (305) un sujet de test au moyen d'une ou plusieurs perturbations physiques ;
    recevoir des données (310) concernant une pluralité de paramètres plus physiologiques d'un sujet de test, en vue d'obtenir une pluralité d'ensembles de données physiologiques ;
    mesurer directement (315) ledit un ou lesdits plusieurs états physiologiques du sujet de test, à l'aide d'un capteur de référence, en vue d'obtenir une pluralité de mesures d'états physiologiques ; et
    corréler (315) les données reçues avec les mesures d'états physiologiques du sujet de test.

2. Procédé selon la revendication 1, dans lequel l'étape consistant à perturber physiquement (215) le corps du patient (110) comprend l'étape consistant à appliquer une pression négative de partie inférieure du corps au corps du patient.

3. Procédé selon la revendication 1, dans lequel l'étape consistant à perturber physiquement (215) le corps du patient (110) comprend l'étape consistant à induire une pression négative sur une voie respiratoire du patient pendant la respiration ou la ventilation.

4. Procédé selon la revendication 3, dans lequel la pression négative est appliquée à l'aide d'un dispositif de seuil d'impédance.

5. Procédé selon la revendication 3, dans lequel la pression négative est appliquée passivement.

6. Procédé selon la revendication 1, dans lequel l'étape consistant à perturber physiquement (215) le corps du patient (110) comprend l'étape consistant à détecter une auto-perturbation du corps.

7. Procédé selon la revendication 1, dans lequel l'étape consistant à perturber physiquement (215) le corps du patient (110) comprend l'étape consistant à induire une pression positive sur au moins une partie du corps du patient.

8. Procédé selon la revendication 7, dans lequel la pression positive est induite à l'aide d'un brassard de prise de tension.

9. Procédé selon la revendication 7, dans lequel l'étape d'induction d'une pression positive sur au moins une partie du corps consiste à induire une pression positive sur une voie respiratoire du patient pendant la respiration ou la ventilation.

10. Procédé selon la revendication 1, dans lequel l'étape consistant à perturber physiquement (215) le corps du patient comprend l'étape consistant à administrer un ou plusieurs médicaments au patient.

11. Procédé selon la revendication 1, dans lequel l'étape consistant à perturber physiquement (215) le corps du patient comprend l'étape consistant à mettre en oeuvre une manoeuvre de valsalva.

12. Procédé selon la revendication 1, dans lequel l'étape consistant à perturber physiquement (215) le corps du patient comprend l'étape consistant à fournir un fluide au patient.

13. Procédé selon la revendication 1, dans lequel l'étape consistant à perturber physiquement (215) le corps du patient comprend l'étape consistant à fournir un courant électrique au patient.

14. Procédé selon la revendication 1, dans lequel les données de diagnostic comprennent :

   - une valeur en cours estimée d'au moins l'un dudit un ou desdits plusieurs états physiologiques du patient ; ou
   - la valeur future prédite d'au moins l'un dudit un ou desdits plusieurs états physiologiques du patient ; ou
   - une valeur future prédite d'au moins l'un dudit un ou desdits plusieurs états physiologiques du patient ; ou
   - une option de traitement recommandée pour au moins l'un dudit un ou desdits plusieurs états physiologiques du patient.

15. Procédé selon la revendication 1, comprenant en outre les étapes ci-dessous consistant à :

   produire une pluralité d'ensembles de données de diagnostic en répétant les opérations consistant à perturber physiquement le corps du patient, à recevoir un ensemble de données d'entrée, à analyser les données d'entrée, et à générer des données de diagnostic ;
   déterminer un changement intervenu dans l'état physiologique du patient, sur la base, au moins en partie, de la pluralité d'ensembles de données de diagnostic ; et
   sevrer le patient, à partir d'un dispositif d'assistance physiologique, sur la base du changement déterminé dans l'état physiologique du patient.

16. Procédé selon la revendication 15, dans lequel le dispositif d'assistance physiologique est sélectionné à partir du groupe constitué par une pompe à ballonnet intra-aortique, un dispositif d'assistance cardiaque, un défibrillateur implantable à synchronisation automatique, un insufflateur, un dispositif d'oxygénation à membrane extracorporelle, un dispositif de pression positive des voies respiratoires, une machine d'anesthésie, un système de soins intensifs intégré, un robot médical, une pompe intraveineuse ou intra-artérielle, une couverture chauffante et une couverture refroidissante.

17. Procédé selon la revendication 1, dans lequel au moins l'un dudit un ou desdits plusieurs capteurs (105) est non invasif.

18. Procédé selon la revendication 1, dans lequel au moins l'un dudit un ou desdits plusieurs capteurs (105) est sélectionné à partir du groupe constitué par un capteur de pression artérielle, un tensiomètre intracrânien, un cathéter de contrôle de la pression veineuse centrale, un électroencéphalographe, un moniteur cardiaque, un capteur Doppler transcrânien, un pléthysmographe d'impédance transthoracique, un oxymètre de pouls, un spectromètre dans le proche infrarouge, un insufflateur, un accéléromètre, un électro-oculogramme, un glucomètre transcutané, un capteur à électrolyte et un stéthoscope électronique.

19. Procédé selon la revendication 1, dans lequel les données d'entrée comprennent des données de forme d'onde de pression artérielle, ou des données de forme d'onde de pléthysmographe.

20. Procédé selon l'une quelconque des revendications précédentes, dans lequel le modèle préexistant fournit un algorithme auquel sont appliquées les données d'entrée, en vue de produire des données de sortie connexes à un état physiologique estimé/prédit du patient.

21. Procédé selon l'une quelconque des revendications précédentes, dans lequel le modèle préexistant est un modèle prédictif d'auto-apprentissage destiné à identifier des corrélations entre un état physiologique et d'autres paramètres physiologiques mesurés.

22. Procédé selon la revendication 21, dans lequel le procédé génère le modèle prédictif de manière autonome.

23. Procédé selon l'une quelconque des revendications précédentes, dans lequel l'étape consistant à corréler les données reçues avec les mesures d'états physiologiques du sujet de test comprend les étapes ci-dessous consistant à :

   identifier un ensemble de signaux le plus prédictif, $S_k$, parmi un ensemble de signaux $s_1, s_2, ..., s_D$, pour chacun d'un ou plusieurs résultats, $o_k$, dans lequel l'ensemble de signaux le plus prédictif, $S_k$, correspond à un premier ensemble de données représentant un premier paramètre physiologique, et dans lequel chaque résultat dudit un ou desdits plusieurs résultats $o_k$ représente une mesure d'état physiologique ;
   effectuer un apprentissage autonome d'un ensemble de modèles probabilistes prédictifs $\hat{o}_k = M_k(S_k)$, où $\hat{o}_k$ est une prédiction de résultat $o_k$ dérivée d'un modèle $M_k$ qui utilise, en qualité d'entrées, des valeurs obtenues à partir de l'ensemble de signaux $S_k$ ; et
   répéter l'opération d'apprentissage autonome de manière incrémentielle à partir de données qui contiennent des exemples de valeurs de signaux $s_1, s_2, ..., s_D$ et de résultats correspondants $o_1, o_2, ..., o_K$.

24. Appareil comprenant :
   un support lisible par ordinateur sur lequel est codé un ensemble d'instructions exécutable par un ou plusieurs ordinateurs pour mettre en oeuvre une ou plusieurs opérations, l'ensemble d'instructions comprenant :

   des instructions pour recevoir, au niveau d'un système informatique (100 ; 500), un ensemble de données d'entrée en provenance d'un ou plusieurs capteurs physiologiques (105), l'ensemble de données d'entrée se rapportant à un ou plusieurs paramètres physiologiques du corps physiquement perturbé d'un patient ;
   **caractérisé par** des instructions pour analyser, avec le système informatique (100 ; 500), les données d'entrée, par rapport à un modèle préexistant, en vue de déterminer la réponse d'un patient à ladite une ou auxdites plusieurs perturbations physiques ;
   des instructions pour générer le modèle préexistant, dans lequel la génération du modèle préexistant comprend les étapes consistant à

      perturber physiquement un sujet de test au moyen d'une ou plusieurs perturbations physiques ;
      recevoir des données concernant une pluralité de paramètres plus physiologiques d'un sujet de test, en vue d'obtenir une pluralité d'ensembles de données physiologiques ;
      mesurer directement ledit un ou lesdits plusieurs états physiologiques du sujet de test, à l'aide d'un capteur de référence, en vue d'obtenir une pluralité de mesures d'états physiologiques ; et
      corréler les données reçues avec les mesures d'états physiologiques du sujet de test ;

   des instructions pour générer des données de diagnostic concernant un ou plusieurs états physiologiques du patient ; et
   des instructions pour afficher, à l'aide d'un dispositif d'affichage, au moins une partie des données de diagnostic.

25. Appareil selon la revendication 24, comprenant en outre :

   un ou plusieurs processeurs (510) ;
   le support lisible par ordinateur en communication avec ledit un ou lesdits plusieurs processeurs, le support lisible par ordinateur présentant l'ensemble d'instructions exécutable, codé sur celui-ci, par le système informatique (100 ; 500), en vue de mettre en oeuvre ladite une ou lesdites plusieurs opérations.

26. Appareil selon la revendication 25, comprenant en outre :
   un dispositif de perturbation (120) configuré de manière à perturber physiquement le corps du patient au moyen d'une ou plusieurs perturbations physiques.

27. Procédé selon la revendication 1,

dans lequel l'étape de perturbation physique (215) du corps du patient (110) comprend l'étape consistant à appliquer une pression négative de partie inférieure du corps au sujet pendant une durée suffisante pour provoquer une variation de la pression artérielle du sujet, mais insuffisante pour provoquer un collapsus cardiovasculaire chez le sujet ;

le procédé comportant l'étape consistant à surveiller des données de forme d'onde de pression artérielle en continu du sujet en utilisant un moniteur non invasif, et notamment à enregistrer une pluralité de lectures de pression artérielle du sujet, la pluralité de lectures de pression artérielle comprenant un premier ensemble d'une ou plusieurs lectures de pression artérielle avant l'application de la pression négative de partie inférieure du corps, et un second ensemble d'une ou plusieurs lectures de pression artérielle pendant ou après l'application de la pression négative de partie inférieure du corps ;

dans lequel l'étape d'analyse (225) consiste à analyser, avec un système informatique (100 ; 500), les données de forme d'onde de pression artérielle par rapport à un modèle préexistant ;

dans lequel l'étape de génération de données de diagnostic consiste à générer, sur la base de l'analyse des données de forme d'onde de pression artérielle, un volume estimé de saignement ou une pression négative de partie inférieure du corps, auquel ou à laquelle le sujet souffrira d'un collapsus cardiovasculaire ; et

dans lequel l'étape d'affichage consiste à afficher des informations sur le volume de saignement auquel le sujet souffrira d'un collapsus cardiovasculaire.

FIG. 1

```
          ┌─────────────────────────────────┐
          │         Generate Model          │◄──────┐
          └─────────────────────────────────┘       │
   205                    │                          │
                          ▼                          │
          ┌─────────────────────────────────┐       │
          │        Monitor Parameters       │◄────┐ │
          └─────────────────────────────────┘     │ │
   210                    │                        │ │
                          ▼                        │ │
          ┌─────────────────────────────────┐     │ │
          │           Perturb Body          │     │ │
          └─────────────────────────────────┘     │ │
   215                    │                        │ │
                          ▼                        │ │
          ┌─────────────────────────────────┐     │ │
          │         Receive Input Data      │     │ │
          └─────────────────────────────────┘     │ │
   220                    │                        │ │
                          ▼                        │ │
          ┌─────────────────────────────────┐     │ │
          │         Analyze Input Data      │     │ │
          └─────────────────────────────────┘     │ │
   225                    │                        │ │
                          ▼                        │ │
          ┌─────────────────────────────────┐     │ │
          │      Generate Diagnostic Data   │     │ │
          └─────────────────────────────────┘     │ │
   230                    │                        │ │
                          ▼                        │ │
          ┌─────────────────────────────────┐     │ │
          │       Display Diagnostic Data   │     │ │
          └─────────────────────────────────┘     │ │
   235                    │                        │ │
                          ▼                        │ │
          ┌─────────────────────────────────┐     │ │
          │    Control Therapeutic Treatment│─────┘ │
          └─────────────────────────────────┘       │
   240                    │                          │
                          ▼                          │
          ┌─────────────────────────────────┐       │
          │     Determine Change in Condition│      │
          └─────────────────────────────────┘       │
   245                    │                          │
                          ▼                          │
          ┌─────────────────────────────────┐       │
          │           Wean Patient          │       │
          └─────────────────────────────────┘       │
   250                    │                          │
                          ▼                          │
          ┌─────────────────────────────────┐       │
          │          Update Model           │───────┘
          └─────────────────────────────────┘
   255
```

FIG. 2

200

```
        ┌─────────────────────────────┐
        │        Perturb Body         │
        └─────────────────────────────┘
   305                │
                      ▼
        ┌─────────────────────────────┐
        │        Receive Data         │
        └─────────────────────────────┘
   310                │
                      ▼
        ┌─────────────────────────────┐
        │ Directly Measure Physiological │
        │            State            │
        └─────────────────────────────┘
   315                │
                      ▼
        ┌─────────────────────────────┐
        │  Correlate Received Data with │
        │      Physiological State     │
        └─────────────────────────────┘
   315
```

FIG. 3                                    300

Collect Data Measurements

405

Derive Signals
From Data Measurements

410

Hypothesize Current or Future
Outcomes

415

Identify Most Predictive Signal
Set for Each Outcome

420

Learn Probabilistic Predictive
Models

425

Learn Predictive Model
Incrementally from Data

430

FIG. 4

400

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060178585 A
- US 20070213619 A
- WO 2010053743 A1, Grudic

### Non-patent literature cited in the description

- **SCHOLKOPF, B. ; SMOLA, A.** Learning with Kernels. MIT Press, 2002
- **WAINWRIGHT, M. J. ; JORDAN, M. I.** Graphical Models, Exponential Families, and Variational Inference. *Foundations and Trends® in Machine Learning,* 2008, vol. 1 (1-2), 1-305
- A formulation for minimax probability machine regression. **STROHMANN, T. ; GRUDIC, G.** Advances in Neural Information Processing Systems. MIT Press, 2003, vol. 15, 769-776
- Sparse greedy minimax probability machine classification. **STROHMANN, T. ; BELITSKI, A. ; GRUDIC, G. ; DECOSTE, D.** Advances in Neural Information Processing Systems. MIT Press, 2004, vol. 16
- **BOHTE, S. ; BREITENBACH, M. ; GRUDIC, G.** Nonparametric classifcation with polynomial mpmc cascades. *International Conference on Machine Learning (ICML),* 2004
- **BREITENBACH, M. ; GRUDIC, G.** Clustering through ranking on manifolds. *International Conference on Machine Learning (ICML),* 2005
- **HASTIE, T. ; TIBSHIRANI, R. ; FRIEDMAN, J.** The Elements of Statistical Learning: Data Mining. Inference, and Prediction. Springer-Verlag, 2009, vol. 2
- **RIPLEY, B. D.** Pattern Recognition and Neural Networks. Cambridge University Press, 1996
- **SCHAPIRE, R. E. ; FREUND, Y. ; BARTLETT, P. ; LEE, W. S.** Boosting the margin: A new explanation for the effectiveness of voting methods. *Annals of Statistics,* 1998, vol. 26 (6), 1651-1686